# EUROPEAN PATENT SPECIFICATION

(11) **EP 3 348 229 B1**
(45) Date of publication and mention of the grant of the patent: **11.03.2020**
(21) Application number: 18156358.6
(22) Date of filing: 15.11.2012
(51) Int. Cl.: A61C 19/05, A61C 9/00, G06F 17/50

(54) **METHOD AND SYSTEM FOR ACQUIRING DATA FROM AN INDIVIDUAL FOR PREPARING A 3D MODEL**
VERFAHREN UND SYSTEM ZUM ERFASSEN VON DATEN EINES INDIVIDUUMS ZUR HERSTELLUNG EINES 3D-MODELLS
PROCÉDÉ ET SYSTÈME POUR ACQUÉRIR DES DONNÉES À PARTIR D'UN INDIVIDU POUR PRÉPARER UN MODÈLE TRIDIMENSIONNEL (3D)

(30) Priority: 15.11.2011 US 201161560117 P
(43) Date of publication of application: 18.07.2018
(62) Divisional of application: 12850614.4
(73) Proprietor: Trispera Dental Inc., Calgary, Alberta T2P 0R3 (CA)
(72) Inventor: COWBURN, George, Calgary, Alberta T3A 2L6 (CA)
(74) Representative: MERH-IP Matias Erny Reichl Hoffmann Patentanwälte PartG mbB

(56) References cited:
- WO-A1-2011/103876

## Description

### CROSS REFERENCE TO RELATED APPLICATIONS

### FIELD

The present disclosure relates generally to acquiring data for structural modeling. More particularly, the present disclosure relates to acquiring data for use in preparing a model of an individual's jaw and arches.

### BACKGROUND

3D modeling of a patient's head has been previously used for tracking jaw movements of the patient. US Patent 7,717,708 to Sachdeva et. al. discloses a method for orthodontic treatment planning. Two or more sets of digital data representing common craniofacial anatomical structures of the patient are obtained from different imaging devices. The data sets may include data representing the external visual appearance of the face of the patient, and data representing a 3D image of the patient's arches. The former may be obtained by a color digital camera, while the latter may be obtained by an optical 3D scanner. The data sets may be superimposed to represent the surface of the patient and interior structures. Software may display a composite 3D representation of craniofacial anatomical structures, and simulate changes in the anatomical position of features such as the jaw, for example during chewing and occlusion. The representation may be used for generating orthodontic appliances.

U.S. Publication 2010/0145898 to Malfliet et al. discloses a method for planning dental treatment. Impressions of the patient's arches are prepared from plaster and scanned. A face bow is used to record a maxillo-mandibular relationship of the patient and a virtual face bow with the same bite registration is created. The virtual face bow, the scanned plaster casts, and 3D images of the patient's face are used to prepare the 3D model. An initial tooth setup is created from library teeth and optimized from that point.

WO2011/103876 discloses a dynamic virtual articulator.

### SUMMARY

Some embodiments disclosed herein obviate or mitigate at least one disadvantage of previous methods of acquiring data useful in preparation of a 3D model.

Modeling mandibular position in three-dimensional space facilitates optimizing diagnostic and treatment capabilities that require accounting for the mandibular path of closure. The vertical, sagittal and frontal parameters are monitored via computerized mandibular scanning instrumentation. The mandible is guided without strain to a myocentric target along the neuromuscular path of trajectory where the jaw musculature is most relaxed.

In a first aspect, the present disclosure provides a method of acquiring data from an individual for preparing a 3D model in accordance with claim 1.

In an embodiment, the third data set is acquired when a selected energy usage value is monitored.

In an embodiment, energy usage is included in the third data set.

In an embodiment, confirming that the maxillo-mandibular relationship is at the physiological rest position comprises exhausting the jaw musculature.

In an embodiment, confirming that the maxillo-mandibular relationship is at the physiological rest position comprises exhausting the jaw musculature by stimulating the jaw musculature to exhaustion.

In an embodiment, confirming that the maxillo-mandibular relationship is at the physiological rest position comprises exhausting the jaw musculature and monitoring energy usage by the jaw musculature, and the third data set is acquired when a selected energy usage value is monitored.

In a further aspect, the present disclosure provides a system for acquiring data for preparing a 3D model from an individual in accordance with claim 8.

In an embodiment, the system further includes a third data acquisition module comprising a third sensor for monitoring energy usage of jaw musculature of the individual, the third data acquisition module is in operative communication with the processor and the processor is configured to cause the second data acquisition module to acquire the third data set when a condition is fulfilled, the condition is a selected energy usage by the jaw musculature, and the selected energy usage is a minimum indicative of the jaw musculature being exhausted and the maxillo-mandibular relationship being at the rest position.

In an embodiment, the system further includes a third data acquisition module comprising a third sensor for monitoring energy usage of jaw musculature of the individual, the third data acquisition module is in operative communication with the processor and the processor is configured to cause the second data acquisition module to acquire the third data set when a condition is fulfilled, the condition is a selected energy usage by the jaw musculature, and the second data acquisition module is stabilized in a data acquisition position where the third data set may be acquired.

In an embodiment, the system further includes a third data acquisition module comprising a third sensor for monitoring energy usage of jaw musculature of the individual, the third data acquisition module is in operative communication with the processor and the processor is configured to cause the second data acquisition module to acquire the third data set when a condition is fulfilled, the condition is a selected energy usage by the jaw musculature, the second data acquisition module is stabilized in a data acquisition position where the third data set may be acquired, the second data set may be acquired from the data acquisition position, and the processor is further configured to cause the second data acquisition module to acquire the second data set.

In an embodiment, the system further includes a third data acquisition module comprising a third sensor for monitoring energy usage of jaw musculature of the individual and wherein the third data set is further acquired by the third data acquisition module.

In an embodiment, the system further includes a muscle exhaustion module for exhausting the jaw musculature.

In an embodiment, the system further includes a third data acquisition module comprising a third sensor for monitoring energy usage of jaw musculature of the individual, and a muscle exhaustion module for exhausting the jaw musculature, the third data acquisition module is in operative communication with the processor, and the processor is configured for causing the second data acquisition module to acquire the third data when energy usage of the jaw musculature is at a minimum energy usage indicative of the jaw musculature being exhausted and the maxillo-mandibular relationship being at the rest position.

In a further aspect, the present disclosure provides a computer readable medium in accordance with claim 16.

Other aspects and features of the present disclosure will become apparent to those ordinarily skilled in the art upon review of the following description of specific embodiments in conjunction with the accompanying figures.

### BRIEF DESCRIPTION OF THE DRAWINGS

Embodiments of the present disclosure will now be described, by way of example only, with reference to the attached figures.
Fig. 1 is a flow chart of a method of acquiring data of an individual with a maxillo-mandibular relationship rest position;
Fig. 2 is a flow chart of the method of Fig. 1 wherein the method further includes acquiring data of the individual with maxillo-mandibular relationships other than the rest position;
Fig. 3 is a schematic of a system for acquiring the data;
Fig. 4 is a schematic of the system of Fig. 3 wherein a first data acquisition module is an intra-oral optical 3D scanner;
Fig. 5 is a schematic of use of the intra-oral scanner of Fig. 4 to acquire the data;
Fig. 6 is a schematic of use of the intra-oral scanner of Fig. 4 to acquire the data;
Fig. 7 is a schematic of the system of Fig. 3 wherein a second data acquisition module is a 3D optical scanner;
Fig. 8 is a schematic of use of the 3D optical scanner of Fig. 7 to acquire the data;
Fig. 9 is a schematic of use of the 3D optical scanner of Fig. 7 to acquire the data;
Fig. 10 is a schematic of use of the 3D optical scanner of Fig. 7 to acquire the data;
Fig. 11 is a schematic of use of the 3D optical scanner of Fig. 7 to acquire the data;
Fig. 12 is a schematic of the system of Fig. 3 wherein the second data acquisition module is a 3D sonographic scanner;
Fig. 13 is a schematic of use of the 3D sonographic scanner of Fig. 12 to acquire the data;
Fig. 14 is a schematic of use of the 3D sonographic scanner of Fig. 12 to acquire the data;
Fig. 15 is a schematic of use of the 3D sonographic scanner of Fig. 12 to acquire the data;
Fig. 16 is a schematic of use of the 3D sonographic scanner of Fig. 12 to acquire the data;
Fig. 17 is a schematic of the system of Fig. 3 further including a third data acquisition module;
Fig. 18 is a schematic of the system of Fig. 17 wherein the third data acquisition module is an electromyograph;
Fig. 19 is a schematic of the system of Fig. 17 wherein the third data acquisition module is in operative communication with the second data acquisition module;
Fig. 20 is a schematic of the system of Fig. 17 wherein the third data acquisition module acquires the data;
Fig. 21 is a schematic of the system of Fig. 3 further including a muscle exhausting module;
Fig. 22 is a flow chart of an embodiment of the method of Fig. 1 further including exhausting the jaw musculature;
Fig. 23 is a schematic of the system of Fig. 21 wherein the muscle exhausting module is a transcutaneous electrical nerve stimulation module;
Fig. 24 is a schematic of the system of Fig. 3 further including the third data acquisition module and the muscle exhausting module;
Fig. 25 is a flow chart of the method of Fig. 1 further including preparing a 3D model from the data;
Fig. 26 is a schematic of the system of Fig. 3 further including a 3D model prepared from the data;
Fig. 27 is a schematic of the components of the 3D model based on the data acquired by the method of Fig. 1;
Fig. 28 is a schematic of the components of the 3D model based on the data acquired by the method of Fig. 2;
Fig. 29 is a schematic of the components of the 3D model based on the data acquired by the method of Fig. 1;
Fig. 30 is a schematic of the components of the 3D model based on the data acquired by the method of Fig. 2;
Fig. 31 is a flow chart of the method of Fig. 25 further including extrapolating to maxillo-mandibular relationships;
Fig. 32 is a schematic of the system of Fig. 26 further including products of manipulation and analysis of the 3D model;
Fig. 33 is a schematic of the method of Fig. 31 wherein the maxillo-mandibular relationship is a centric occlusion position; and
Fig. 34 is a schematic of the system of Fig. 32 wherein the products of manipulation and analysis of the 3D model include a centric occlusion position.

### DETAILED DESCRIPTION

Some individuals lack sufficient dentition to define a natural occlusal position. In these individuals, a habitual occlusal position ("**habitual position**") may be defined over time. Where the habitual position is established, it is a convenient reference point when planning dental treatment. As a result, the habitual position is sometimes used as a starting point for restoration of a single tooth with a crown or filling or even a quadrant of dental restorations. However, the habitual position is not necessarily an optimal stable neuromuscular occlusion. Basing a set of dentures on the habitual position does not necessarily facilitate optimization of mandible to cranium relationship, optimal facial cosmetics, or optimal dental aesthetics (tooth morphology and dental architecture).

Long-term patient comfort and muscular balance of the complete posturing system (which includes the head, the mandible, the cervical region of the neck, the shoulder and pelvis and legs) may be adversely affected by an inappropriately established bite caused by a dental appliance. It is, therefore, desirable to provide a preferable starting position for design of dental appliances.

In some previous methods, jaw tracking is based on observation of intra-oral objects during jaw movement. This may require that the lips and cheeks to be moved to provide a clear view of the objects. Cheek retractors are often used to spread the subject's lips and provide visibility of the objects. Use of cheek retractors necessarily affects the musculature of the patient and may stress the temporo-mandibular joint by forcing the mouth to open widely.

Generally, the present disclosure provides a method and system for acquiring data from which a 3D model of an individual's head may be prepared. As used herein, the expression "3D model of an individual's head" includes a 3D model of only a portion of the individual's head, including but not limited to at least a portion of each of the individual's mandible, maxilla, maxillary arch, and mandibular arch. The method includes, and the system facilitates, acquiring data of the individual's maxillo-mandibular relationship when the individual's jaw is at a physiological rest position ("**rest position**"). A 3D model prepared from the data provides an accurate representation of the individual's maxillo-mandibular relationship at the rest position, as the data is acquired when the maxillo-mandibular relationship is at the rest position (in contrast with acquiring data at a different position and extrapolating to the rest position). The individual's actual rest position determines that of the 3D model. The rest position of the 3D model thereby accounts for the interrelationship of all the entities within the stomatognathic system, including joints, muscles, nerves, gums, implants (if any), and teeth (if any), which affect the rest position. A 3D model prepared without any data of an individual at rest position is less likely to reliably distinguish a rest position from a habitual position, or other position.

The 3D model facilitates accurate determination of other potentially useful maxillo-mandibular relationships. For example, the 3D model may be applied to jaw tracking and extra-oral bite assessment of individuals lacking sufficient dentition to establish a bite, for example edentulous individuals. The data may facilitate determination of a natural position at which centric occlusion ("**CO**"; which occurs when an individual's teeth are at maximum intercuspation, and the individual's jaw is at a "**CO position**") would occur if the individual had sufficient dentition to establish a bite. The data may thus facilitate approximation of an optimal neuromuscular CO position. An estimated CO position may be applied to preparing dentures for individuals who do not have enough teeth to define a bite.

It is common for a denturist or other dental professional to establish a CO position when preparing an appliance. Where the individual lacks sufficient dentition to establish the CO position, extrapolation is necessarily required to determine an appropriate maxillo-mandibular relationship in which CO should occur with an appliance. An edentulous individual will lack sufficient dentition to establish the CO position. Some partially dentate individuals will also lack sufficient dentition to establish CO, for example individuals with incisors but no molars.

Establishing a CO position based on the rest position when preparing an appliance may facilitate improvement and optimization of resulting dental function, stability, and harmony, of the stomatognathic system including the appliance. Establishing the CO position based on the rest position may also facilitate one or more of the following:
- optimization of the individual's occlusal scheme to a normal occlusal scheme where a normal occlusal scheme will provide appropriate functionality to the individual, or accounting for any jaw relationship classification or malocclusion where the individual's CO position may require as much;
- optimization of dental aesthetics (including tooth shape, contour, anatomy and morphology in both the anterior and posterior regions);
- optimization of facial cosmetics due to a more harmonious muscular balance when an optimal physiologic mandibular position is found; or
- mitigation of possible musculoskeletal occlusal signs and symptoms including: headaches, ear congestion feelings, ringing in the ears, pressure behind the eyes, teeth sensitivities, temporomandibular joint noise, masticatory muscle tenderness, neck and shoulder pain.

### Rest Position

The rest position is a position of the mandible in space relative to the maxilla (vertical, anterior-posterior, and lateral relative to the head in an upright postural position) along an isotonic path of mandibular closure. At the rest position, jaw musculature, including the extensor and depressor muscles that move the mandible, is postured at a position wherein it exerts a minimum of electrical activity. Expenditure of energy by the jaw musculature required to maintain the rest position is minimal compared to other positions along a path of mandible hinging. In the rest position, the individual's condyles are in a neutral, unrestrained position.

The rest position of an individual can be determined with reference to the individual. The rest position cannot be determined on a mechanical device that simulates mandibular movements, such as a dental articulator. A mandibular position, or maxillo-mandibular relationship, can be influenced by factors including postural problems of the head, neck cervical region, and back region. Internal derangements of the temporomandibular joint, emotional factors and systemic health factors of the individual can also contribute to a compromised mandibular position. It is generally beneficial to account for these factors before establishing a rest position. In some cases, failure to account for these factors results in an erroneous rest position. For example, a factor may have to be addressed or removed before establishing a rest position, which may be used to extrapolate to a bite registration. In another example, a factor may further complicate extrapolating rest position from other positions, increasing an advantage to acquisition of data of the individual at rest position.

The rest position is a true rest position, in contrast with a habitual position. The habitual position is an acquired maxillo-mandibular position that may be anteriorly positioned along the condylar translation pathway. In a given individual, the rest position and the habitual position may coincide or be very close. However, the energy required by jaw musculature to maintain the habitual position is not necessarily a minimum as is the rest position. The habitual position is sometimes used as a starting point in determining a CO position in edentulous individuals. However, beginning with the habitual position may provide a less desirable outcome with respect to planning dental treatment than beginning with the rest position.

For simplicity and clarity of illustration, reference numerals may be repeated among the figures to indicate corresponding or analogous elements. Numerous details are set forth to provide an understanding of the examples described herein. The examples may be practiced without these details. In other instances, well-known methods, procedures, and components are not described in detail to avoid obscuring the examples described. The description is not to be considered as limited to the scope of the examples described herein.

### Method

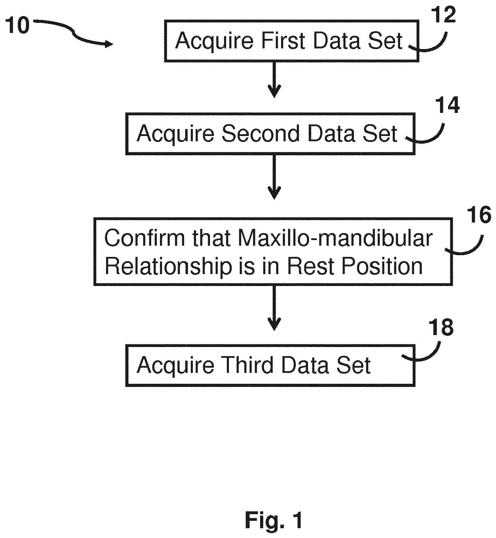

Fig. 1 is a flow chart of a method 10 of acquiring data from an individual. The method includes acquiring a first data set 12, acquiring a second data set 14, confirming that the maxillo-mandibular relationship of the individual is at rest position 16, and acquiring a third data set 18. The first data set facilitates modeling of the individual's maxillary and mandibular arches. The arches each include tissue (for example gingival tissue, soft tissue, or keratinized tissues) and structures (for example prosthetics and natural dentition). The arches each include alveolar ridges (also called processes or margins). In a maxillary-edentulous individual with no maxillary prosthetics, the maxillary arch would include the palate and the maxillary residual ridge only. In a mandibular edentulous individual with no mandibular prosthetics, the mandibular arch would include the mandibular residual ridge only.

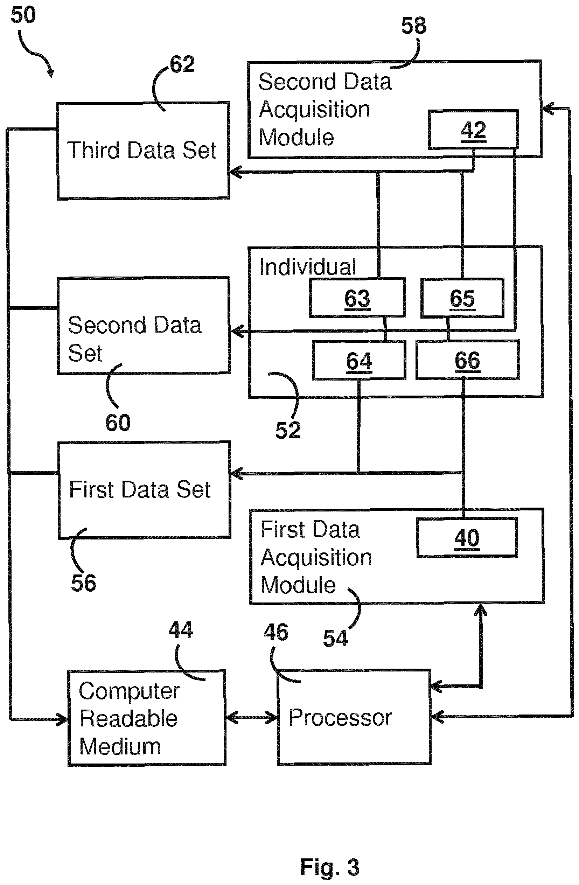

The second data set relates the maxillary arch to the maxilla, and the mandibular arch to the mandible. For example, the arches may be related to external features of the individual's maxilla and mandible. Alternatively, the arches may be related to the tissue or bone structure of the maxilla and mandible. Prior to acquiring the third data set 18, the maxillo-mandibular relationship is confirmed to be in the rest position 16. The third data set is acquired while the maxillo-mandibular relationship is at the rest position. The third data set facilitates modeling of the individual's head based on empirical data of the maxillo-mandibular relationship at rest position. The individual, the first data set, the second data set, the third data set, the maxillary arch, the mandibular arch, the maxilla, and the mandible are shown schematically in the below system figures (e.g. Figs. 3, 5, 6, etc.). The method 10 may be practiced with different orders for the individual portions of the method 10. The same applies to other methods disclosed herein except where specified otherwise.

In some embodiments, the maxillo-mandibular relationship may be in the rest position or close to the rest position when the second data set is acquired.

In some embodiments, the maxillo-mandibular relationship is confirmed to be in rest position by observing the individual moving their jaw in specific ways, for example observing the individual while the individual relaxes their jaw, licks their lips, or swallows.

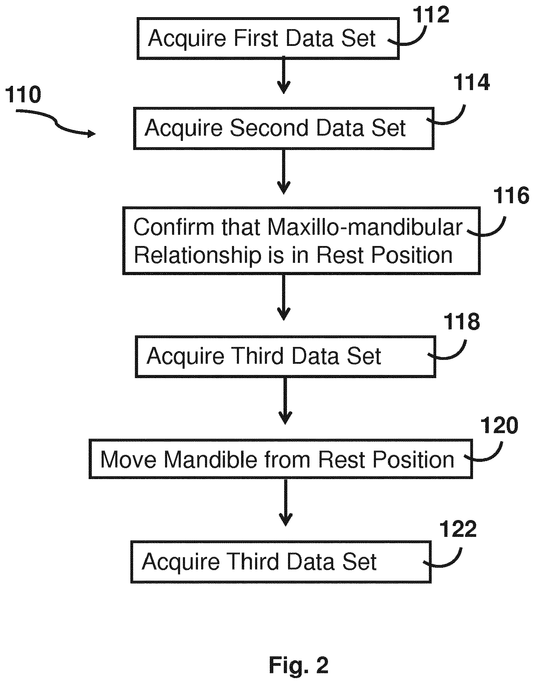

Fig. 2 a method 110 including changing the maxillo-mandibular relationship from the rest position 120, and acquiring a portion of the third data set 122 at the maxillo-mandibular relationship other than the rest position. For example, moving the mandible from the rest position 120 may be done by the individual or by a clinician. Moving the mandible from the rest position 120 may include moving the mandible to a position where the mandible is translated left or right, extruded, retruded, or hingedly moved from the rest position, or any combination thereof. In an embodiment, portions of the third data set may be acquired in real time. For example, portions of the third data set may be acquired while the maxillo-mandibular relationship changes.

### System

Fig. 3 is a schematic of a system 50 for acquiring data from an individual 52. The individual 52 has a maxilla 63, a maxillary arch 64, mandible 65, and a mandibular arch 66. The system 50 includes a first data acquisition module 54 for acquiring a first data set 56, and a second data acquisition module 58 for acquiring a second data set 60 and a third data set 62. One example of the first data acquisition module 54 and two examples of the second data acquisition module 58 are provided below. However, devices other than those exemplified which are capable of acquiring the first data set 56, the second data set 60, and the third data set 62 are substitutable with the examples provided herein. The second data set 60 and the third data set 62 may each be acquired with either of the examples provided of the second data acquisition module 58 and using a single second data acquisition module 58 may lower the cost of the system. Alternatively, the second data set 60 and the third data set 62 may each be acquired with distinct second data acquisition modules 58. Where two second data acquisition modules 58 are used, each second data acquisition module 58 may be an example of the same type of device, or each second data acquisition module 58 may be a different device. Alternatively, other examples of the first data acquisition module 54 and the second data acquisition module 58 may be used in combination with the examples provided herein, in combination with each other, or both.

The first data acquisition module 54 includes a first sensor 40 for acquiring the first data set 56 from the maxillary arch 64, and from the mandibular arch 66. The second data acquisition module 58 includes a second sensor 42 for acquiring the second data set 60 from the maxilla 63 the maxillary arch 64, and from the mandible 65 and the mandibular arch 66. The second sensor 42 is also for acquiring the third data set 62 from the maxilla 63 and the mandible 65. The system 50 includes a computer readable medium 44, for example a transitory or non-transitory computer readable medium, for storing the first data set 56, the second data set 60, and the third data set 62. The first data acquisition module 54 is in operative communication with the computer readable medium 44 for storing the first data set 56. The second data acquisition module 58 is in operative communication with the computer readable medium 44 for storing the second data set 60 and the third data set 62. In some embodiments, the first data acquisition module 54 may be in operative communication with the computer readable medium 44 through a wireless or wired connection. In some embodiments, the second data acquisition module 58 may be in operative communication with the computer readable medium 44 through a wireless or wired connection.

The system 50 includes a processor 46 for controlling the first data acquisition module and the second data acquisition module. The processor 46 is also accessing the computer readable medium 44 and the first data set 56, the second data set 60, and the third data set 62 stored on the computer readable medium 44. In some embodiments, the first data acquisition module 54, the second data acquisition module 58, or both, may be used with associated software that is executed by the processor 46. In some embodiments, the first data acquisition module 54 may be in operative communication with the processor 46 through a wireless or wired connection. In some embodiments, the second data acquisition module 58 may be in operative communication with the processor 46 through a wireless or wired connection.

In some embodiments, the first data acquisition module 54 may include additional features beyond the first sensor 40, for example a first onboard processor, or a first onboard computer readable medium and a first onboard processor. In some embodiments, the first data set 56 may be stored on the first onboard computer readable medium for transfer to the computer readable medium 44. Alternatively, the first onboard computer readable medium may serve as the computer readable medium 44. In some embodiments the first onboard processor is used with associated software, and the associated software may be executed by the first onboard processor, the processor 46, or both.

In some embodiments, the second data acquisition module 58 may include additional features beyond the second sensor 42, for example a second onboard processor, or a second onboard computer readable medium and a second onboard processor. In some embodiments, the second data set 60 may be stored on the second onboard computer readable medium for transfer to the computer readable medium 44. Alternatively, the second onboard computer readable medium may serve as the computer readable medium 44. In some embodiments the second onboard processor is used with associated software, and the associated software may be executed by the second onboard processor, the processor 46, or both.

The first data set 56 includes features of each of the maxillary arch 64 and the mandibular arch 66. The first data set 56 facilitates modeling of the maxillary arch 64 and the mandibular arch 64. The maxillo-mandibular relationship of the individual 52 is not relevant during acquisition of the first data set 56. Any suitable method may be used to provide access to the maxillary arch 64 and the mandibular arch 66 by the first data acquisition module 54, for example cheek retractors.

The second data set 60 includes features of the maxillary arch 64 and the maxilla 63, and of the mandibular arch 66 and the mandible 65. The second data set 60 facilitates establishing a relationship between the maxillary arch 64 and the maxilla 63, and between the mandibular arch 66 and the mandible 65. The second data set 60 includes data relating to at least a portion of the maxillary arch 64 and at least a portion of the maxilla. The second data set 60 also includes data relating to at least a portion of the mandibular arch 66 and at least a portion of the mandible 65.

In some cases, the individual 52 may not be able to provide access to both the maxillary arch 64 and the mandibular arch 66 by the second data acquisition module 58 simultaneously. In such cases, the second data acquisition module 58 may be used to acquire a first portion of the second data set 58, the individual 52 may then be repositioned, and the second data acquisition module 58 may be used to acquire a second portion of the first data set 56. For example, the first portion of the second data set 58 may include at least a portion of the maxillary arch 64 and at least a portion of the maxilla 63. Similarly, the second portion of the second data set 58 may include at least a portion of the mandibular arch 66 and at least a portion of the mandible 65. The maxillo-mandibular relationship need not be in the rest position, or any other particular position, when acquiring the second data set 58.

The third data set 62 includes the maxilla 63 and the mandible 65 when the maxillo-mandibular relationship is at the rest position. The third data set 62 facilitates modeling of the individual's head at rest position. The third data set 62 need not include data of the maxillary arch 64 or the mandibular arch 66.

Two or more of the data sets may be acquired simultaneously. For example, where a single data set of the individual 52 at rest position including at least a portion of the maxilla 63, the maxillary arch 64, the mandible 65, and mandibular arch 66 may be acquired, the single data set may serve as each of the second data set 60 and the third data set 62. Alternatively, if a single data set of the individual 52 at rest position including sufficient data to model the maxillary arch 64 and the mandibular arch 66, to relate the maxilla 63 to the maxillary arch 64, to relate the mandible 65 to the mandibular arch 66, and including at least a portion of the maxilla 63 and mandible 65 in the same data point, can be acquired, the single data set may serve as each of the first data set 56, the second data set 60, and the third data set 62.

### First Data Acquisition Module - Optical 3D Intra-Oral Scanner

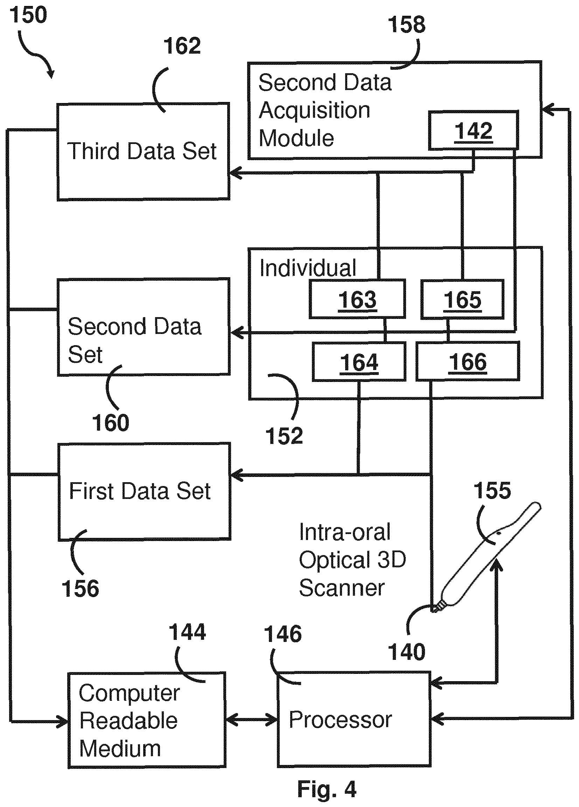

Fig. 4 is a schematic of a system 150 wherein the first data acquisition module is an optical 3D intra-oral scanner 155. The intra-oral scanner 155 and associated software are used to scan the maxillary arch 164 and the mandibular arch 166 to acquire the first data set 156. Examples of intra-oral scanners suitable for the system 150 include the a.tron3D gmbh bluescan-I™ 3D intraoral scanner, the Planmeca Oy PlanScan™ digital impression scanner, the Sirona CEREC Omnicam, the Slrona CEREC Bluecam, the Cadent iTero™ digital impression system, and the 3M™ True Definition Scanner.

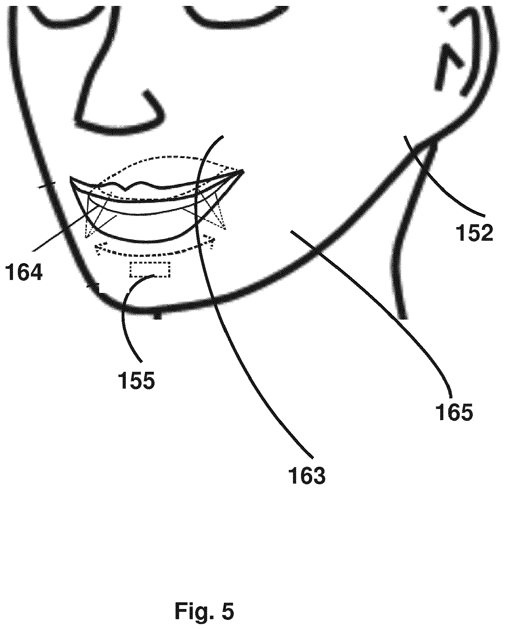

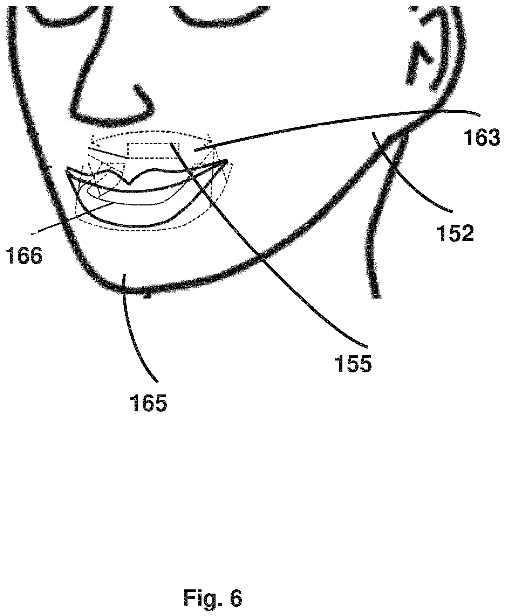

Figs. 5 and 6 are schematics of use of the intra-oral scanner 155 in an embodiment of the method 10 to acquire the first data set 156. In Fig. 5, the intra-oral scanner 155 is used to scan the maxillary arch 164, acquiring a first portion of the first data set 156. In Fig. 6, the intra-oral scanner is used to scan the mandibular arch 166, acquiring a second portion of the first data set 156.

### Second Data Acquisition Module - Extra-Oral 3D Optical Scanner

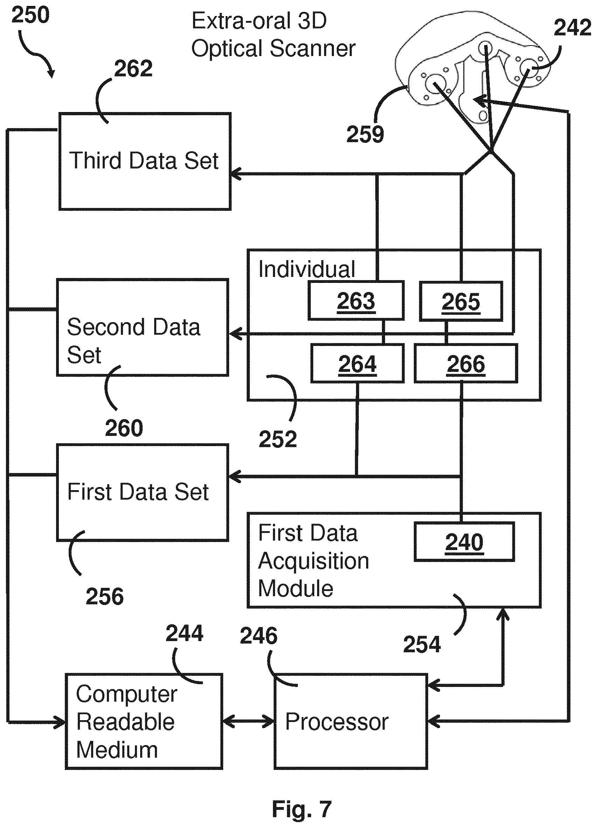

Fig. 7 is a schematic of a system 250 wherein the second data acquisition module is a 3D optical scanner 259 for scanning the individual's head from a perspective outside of their mouth ("**extra-oral scanner**") to acquire the second data set 260 and the third data set 262. Examples of extra-oral scanners include the Creaform Inc. VIUscan™ color laser scanner, the Northern Digital Inc. VircaSCAN™ handheld 3D laser scanner, and structured white light imaging scanners such as the 3D3 Solutions HDI Advance 3D Scanner. In an embodiment, the 3D optical scanner 259 may be used to acquire the first data set 256 set by taking impressions of the maxillary arch 264 and of the mandibular arch 266, then scanning the impressions with the 3D optical scanner 259. A cast could be made from the impressions and the cast scanned with the 3D optical scanner 259 to acquire the first data set 256

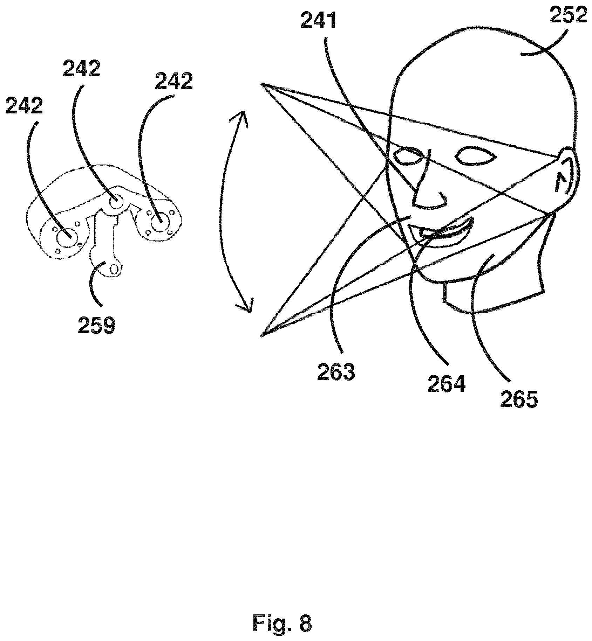

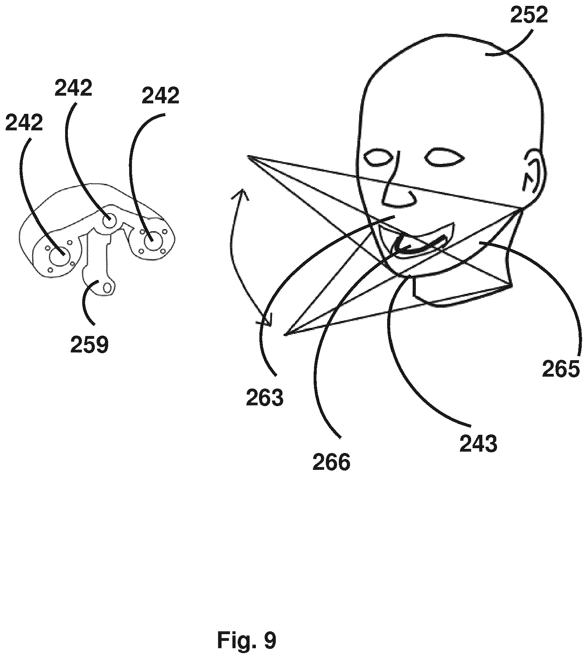

Figs. 8 and 9 are schematics of use of the extra-oral scanner 259 of the system 250 in an embodiment of the method 10 to acquire the second data set 260. Whether the extra-oral scanner 259 has a clear line of sight to the maxillary arch 263, the mandibular arch 264, or both, may be determined by, for example, the maxillo-mandibular relationship, and whether the lips of the individual 252 are retracted. A first portion of the second data set 260 is acquired in Fig 8. A second portion of the second data set 260 is acquired in Fig. 9.

In Fig. 8, the extra-oral scanner 259 is used to scan at least a portion of the maxilla 263 and at least a portion of the maxillary arch 264, acquiring the first portion of the second data set 260. The portion of the maxilla 263 includes a reference point, for example a nasiun 241. The reference point should be relatively stable with respect to the maxillary arch 264 at different maxillo-mandibular relationships.

In Fig. 9, the extra-oral scanner 259 is used to scan at least a portion of the mandible 265 and at least a portion of the mandibular arch 266, acquiring a second portion of the second data set 260. The portion of the maxilla 265 includes a reference point, for example an inferior aspect of the mental protuberance 243. The reference point should be relatively stable with respect to the mandibular arch 266 at different maxillo-mandibular relationships.

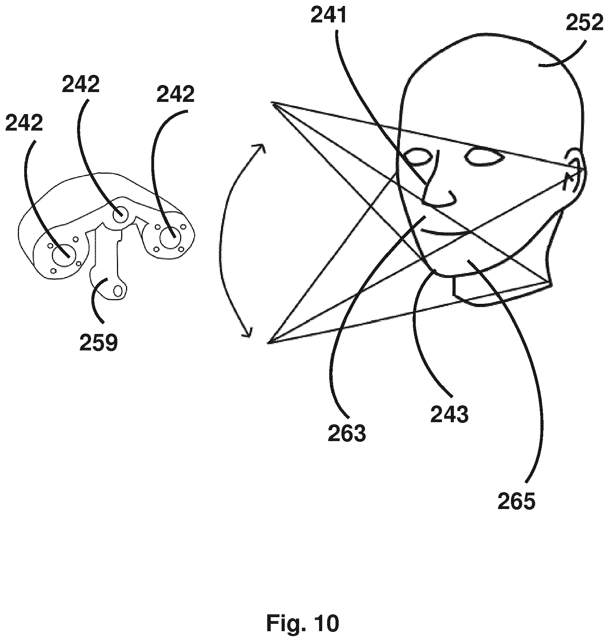

Fig. 10 is a schematic of use of the extra-oral scanner 259 of the system 250 in an embodiment of the method 10 to acquire the third data set 262. The maxilla 263 and the mandible 265 are both scanned when the maxillo-mandibular relationship is at the rest position. Selected reference points on the maxilla 263 and the mandible 265 are each included in the third data set 262. The reference points may for example include the nasiun 241 and the inferior aspect of the mental protuberance 243. In an embodiment, the extra-oral scanner 259 may be stabilized in a data acquisition position where the third data set 262 may be acquired without a clinician in the room during acquisition of the third data set 262.

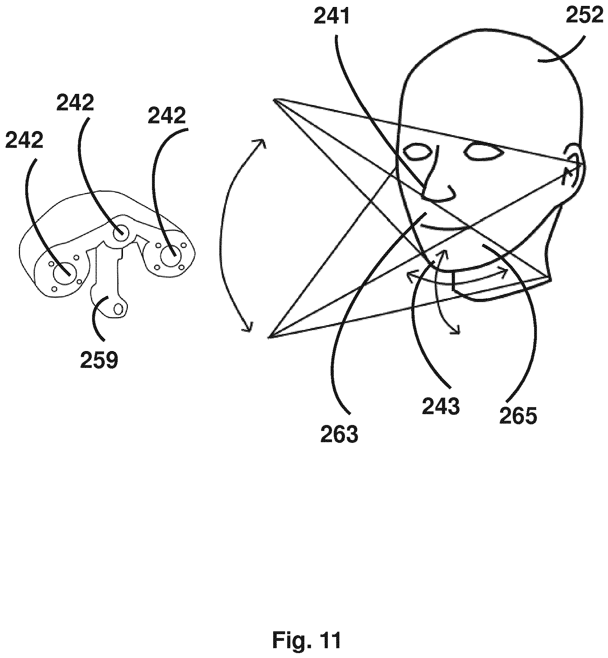

Fig. 11 is a schematic of use of the extra-oral scanner 259 of the system 250 in an embodiment of the method 110 to acquire the third data set 262. The maxilla 263 and the mandible 265 are each scanned when the maxillo-mandibular relationship is at the rest position to acquire a first portion of the third data set 262 (see Fig. 10). In addition, the maxilla 263 and the mandible 265 are each scanned when the mandible 265 is moved from the rest position. For example, the mandible 265 may be translated left or right, extruded, retruded, or hinged from the rest position. Selected reference points on the maxilla 263 and the mandible 265 are each included in the third data set 262. The reference points may for example include the nasiun 241 and the inferior aspect of the mental protuberance 243. In an embodiment, the extra-oral scanner 259 may be stabilized in a data acquisition position where the third data set 262 may be acquired without a clinician in the room during acquisition of the third data set 262.

### Second Data Acquisition Module - 3D Sonographic Scanner

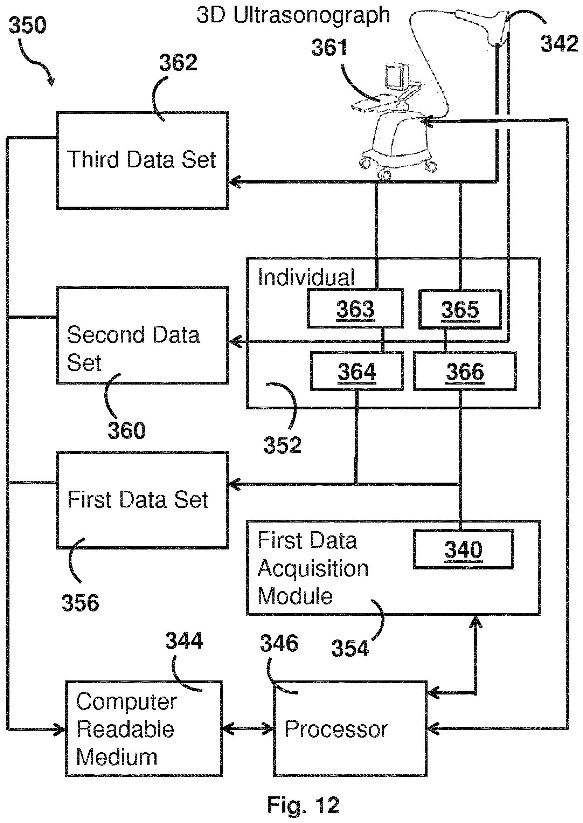

Fig. 12 is a schematic of a system 350 wherein the second data acquisition module is a 3D sonographic scanner 361. The 3D sonographic scanner 361 may be used to acquire the second data set 360 and the third data set 362.

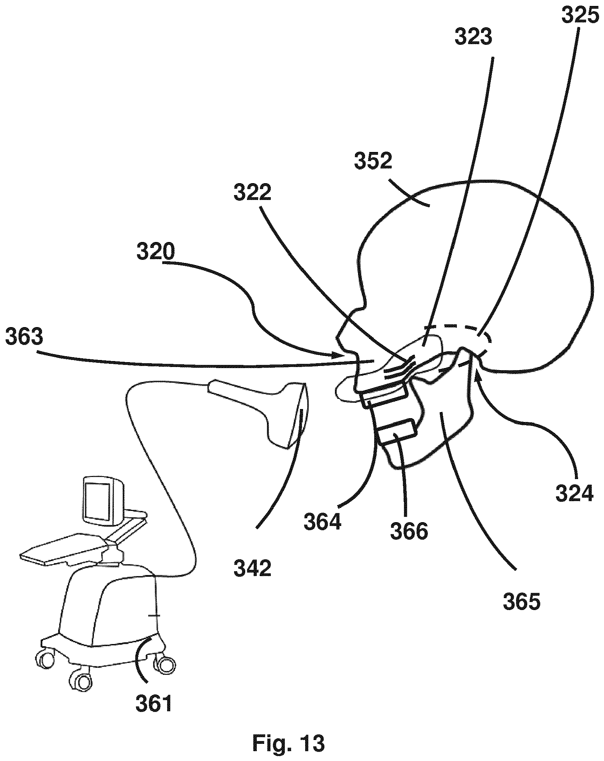

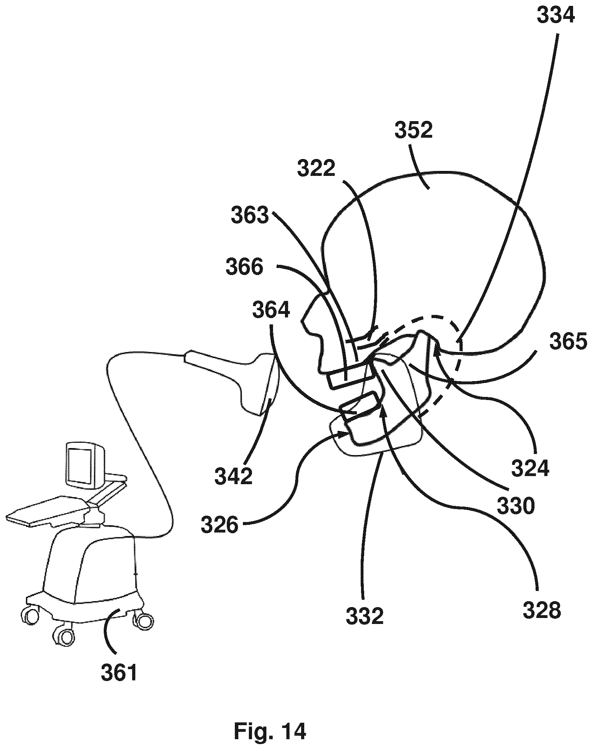

Figs. 13 and 14 are schematics of use of the 3D sonographic scanner 361 in an embodiment of the method 10 to acquire the second data set 360. A first portion of the second data set 360 is acquired in Fig. 13. A second portion of the second data set 360 is acquired in Fig. 14.

In Fig. 13, the 3D sonographic scanner 361 is used to acquire data of at least a portion of the maxillary arch 364 and at least a portion of the maxilla 363, the portion of the maxilla 363 being in the same field of view for the sensor 342 as at least a portion of the mandible 365 at the rest position. For example, data acquisition may begin at an anterior midline 320 of the maxilla 363, following the maxillary arch 364 towards a zygomatic arch 322, acquiring the first portion of the second data set 360 for modeling a first maxillary area 323 including at least a portion of the maxillary arch 364 and at least a portion of the zygomatic arch 322. Alternatively, data acquisition may begin at the anterior midline 320 of the maxilla 363 and follow the maxillary arch 364 towards a temporomandibular joint 324 ("**TMJ**"), acquiring the first portion of the second data set 360 for further modeling a second maxillary area 325 including at least a portion of the maxillary arch 364 and at least a portion of the TMJ 324.

In Fig. 14, the 3D sonographic scanner 361 is used to image at least a portion of the mandibular arch 364 and at least a portion of the mandible 365, the portion of the mandible 365 being in the same field of view for the sensor 342 as at least a portion of the maxilla 363 at the rest position. For example, data acquisition may begin at an anterior midline 326 of the mandible 365 and proceed distally following the mandibular arch 366, past a retromolar pad region 328, and to a coronoid process 330, acquiring the second portion of the second data set 360 for modeling a first mandibular area 332 including at least a portion of the mandibular arch 366 and at least a portion of the coronoid process 330. Full opening of the mandible 365 may facilitate acquisition of data relating to a larger portion of the coronoid process 330, unobscured by the zygomatic arch 322. Alternatively, data acquisition may begin at the anterior midline 326 of the mandible 365 and proceed distally following the mandibular arch 366, past the retromolar pad region 328, past the coronoid process 330, and to the TMJ 324, acquiring the second portion of the second data set 360 for further modeling a second mandibular area 334 including at least a portion of the mandibular arch 366 and at least a portion of the TMJ 324.

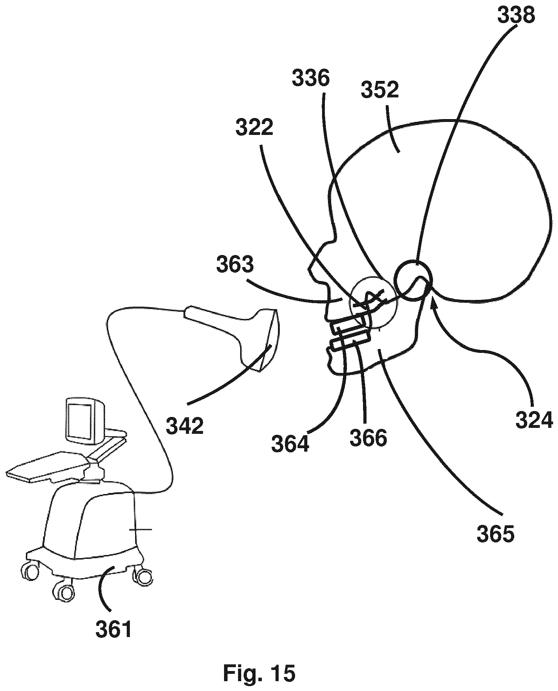

Fig. 15 is a schematic of use of the 3D sonographic scanner 361 in an embodiment of the method 10 to acquire the third data set 362. In the rest position, the third data set 362 is acquired of at least a portion of each of the maxilla 363 and mandible 365. For example, data of each of the zygomatic arch 322 and the coronoid process 330 may be acquired by acquiring the third data set 362 at a zygomatic arch area 336. Alternatively, data of the TMJ 324 may be acquired acquiring the third data set 362 at a TMJ area 338.

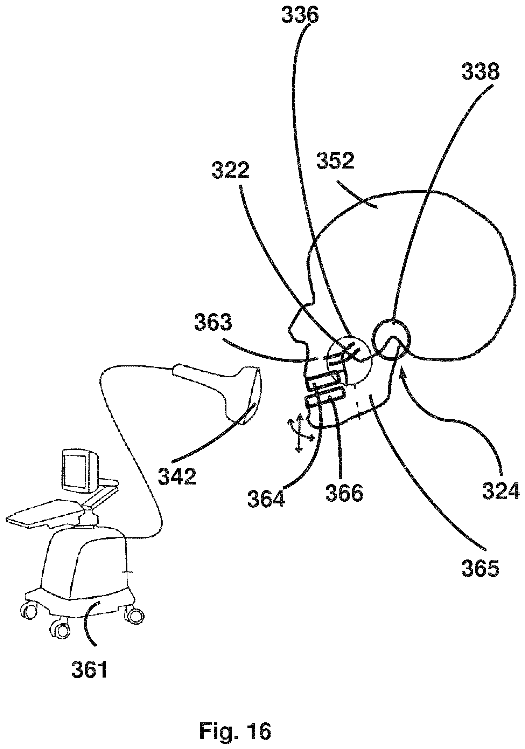

Fig. 16 is a schematic of use of the 3D sonographic scanner 361 in an embodiment of the method 110 to acquire the third data set 362. In addition to rest position, the third data set 362 may be acquired with the mandible 365 translated left or right, extruded, retruded, or hingedly moved from the rest position. In an embodiment, the third data set 362 may include movement of the zygomatic arch 322 relative to the coronoid process 330 in real time to track movements of the mandible 365. Alternatively, the third data set 362 may include movement of the TMJ 324 in real time to track movements of the mandible 365.

In an embodiment, when acquiring the third data set 362, 3D sonography may be applied to the maxilla 363 and mandible 365 unilaterally. One example of the second sensor 342 would be used to acquire a first portion of the third data set 362 in respect of the zygomatic arch area 336 on the left side of the individual 352, and the same example of the second sensor 342 would then be used to acquire a second portion of the third data set 362 in respect of the zygomatic arch area 336 on the right side of the individual 352. Alternatively, one example of the second sensor 432 would be used to acquire the third data set 362 in respect of the zygomatic arch area 336 on the right or the left side only of the individual 352, and not on both sides. Alternatively, one example of the second sensor 342 would be used to acquire a first portion of the third data set 362 in respect of the TMJ area 338 on the left side of the individual 352, and the same example of the second sensor 342 would then be used to acquire a second portion of the third data set 362 in respect of the TMJ area 338 on the right side of the individual 352. Alternatively, one example of the second sensor 432 would be used to acquire the third data set 362 in respect of the TMJ area 338 on the right or the left side only of the individual 352, and not on both sides.

In an embodiment, when acquiring the third data set 362, 3D sonography may be applied to the maxilla 363 and mandible 365 bilaterally simultaneously. Two examples of the second sensor 342 would be used to simultaneously acquire the third data set 362 in respect of the zygomatic arch area 336 on the left side of the individual 352 and the zygomatic arch area 336 on the right side of the individual 352. Alternatively, two examples of the second sensor 342 would be used to simultaneously acquire the third data set 362 in respect of the TMJ area 338 on the left side of the individual 352 and the TMJ area 338 on the right side of the individual 352. Acquiring data bilaterally may increase the quality of the third data set 362, for example where maxillo-mandibular relationships other than the rest position are included in the third data set 362, and where the maxillo-mandibular relationships other than the rest position include lateral movement of the mandible 365 relative to the maxilla 363.

In an embodiment, the 3D sonographic scanner 361 may be stabilized in a data acquisition position where the third data set 362 may be acquired of at least a portion of the maxilla 363 and at least a portion of the mandible 365 when the individual 352 is in the rest position. Stabilization of the 3D sonographic scanner 361 in place removes the requirement for having a clinician in the room during acquisition of the third data set 362. For example, the 3D sonographic scanner 361 may be stabilized in a data acquisition position to facilitate acquiring data of the zygomatic arch area 336. Alternatively, the 3D sonographic scanner 361 may be stabilized in a data acquisition position to facilitate acquiring data of the TMJ area 338. In some embodiments, one example of the second sensor 342 would be stabilized in the data acquisition position, and the individual 352 would be scanned once on their left side and once on their right side. In some embodiments, two examples of the second sensor 342 would be stabilized in the data acquisition position, with a first example of the second sensor 342 on the left side of the individual 352 and a second example of the second sensor 342 on the right side of the individual 352.

### Confirming Rest Position

Methods of confirming whether an individual is in the rest position are known among dental professionals, and any suitable method may be applied. Simple methods such as requiring the individual to say "Emma", chew, or retrude their mandible may be suitable. More objective and reproducible methods of determining rest position are more suitable to determining when to acquire data for preparing a model of the individual at the rest position. The more objective and reproducible methods of determining rest position are more suitable to determining when to acquire the data, as the habitual position and the rest position may often be close.

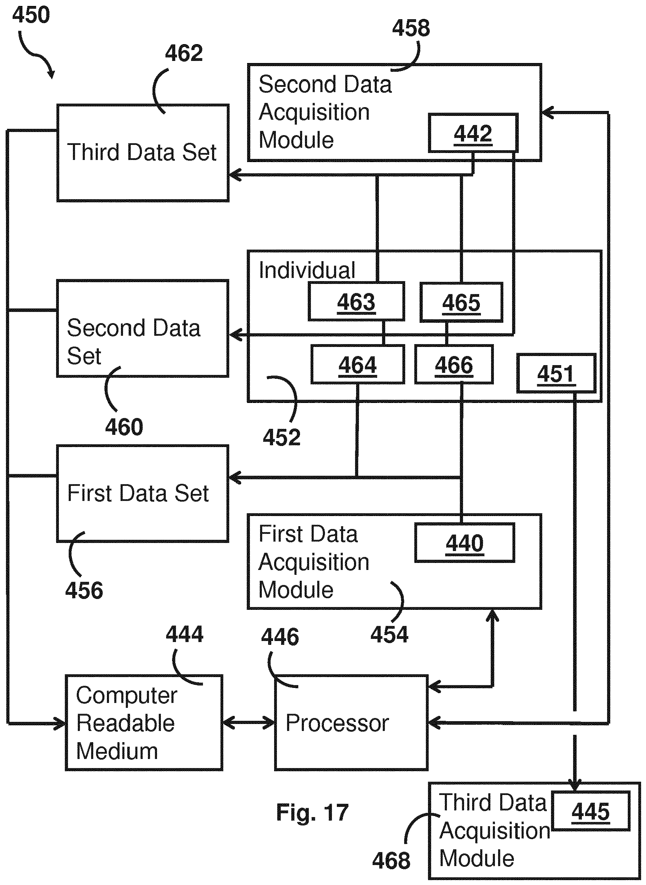

Fig. 17 is a schematic of a system 450 further including a third data acquisition module 468. The third data acquisition module 468 includes a third sensor 345 for acquiring data relating to energy used by jaw musculature 451 of the individual 452. Any suitable technique for acquiring data of energy usage by the jaw musculature 451 may be used. The data of energy usage by the jaw musculature may be presented to a user, for example the absolute or relative level of energy usage by the jaw musculature 451 as a function of the maxillo-mandibular relationship. The data may be presented to the user on a visual display, such as an LCD display, or alternatively through an aural, tactile, or other feedback medium. This allows a user of the system 450 to determine at which maxillo-mandibular relationship the energy usage is at a selected value. The selected value may be a minimum, which is equated to the rest position. In an embodiment, the method 10 includes confirming that the amount of energy used by the jaw musculature 451 to remain in position has reached a minimum.

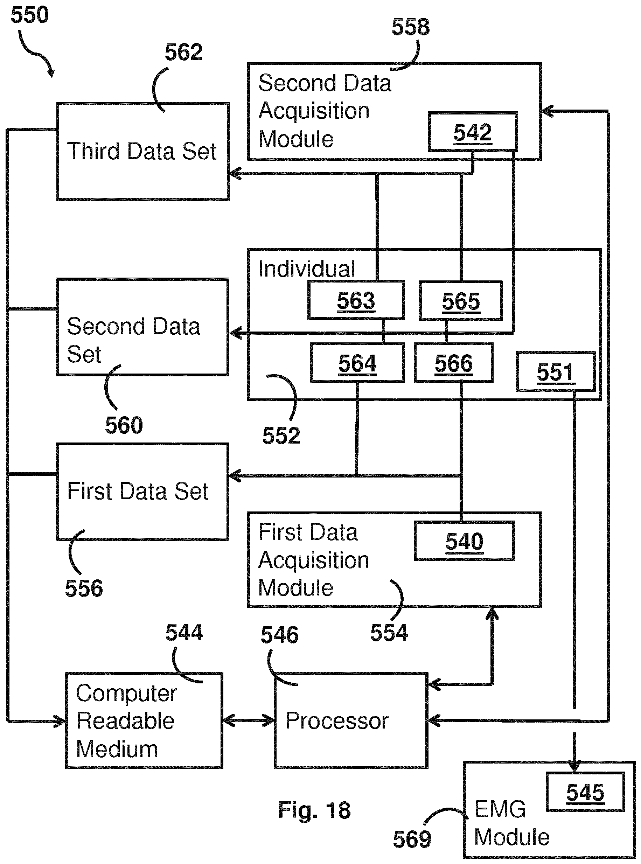

Fig. 18 is a schematic of a system 550 wherein the third data acquisition module is an electromyography ("**EMG**") module 569. The EMG module 569 may monitor the electric potential of the jaw musculature 551 by EMG at different maxilla-mandibular relationships, providing data of energy usage by the jaw musculature 551 as a function of the maxillo-mandibular relationship. The specific mucles in the jaw musculature 551 targeted by the EMG module 569 include masseter muscles and anterior temporalis muscles. In the EMG module 569, the third sensor 545 may be bipolar surface electrodes, which allow surface EMG data to be acquired from multiple muscle sites simultaneously and in real time. Software executed by the processor 546 allows acquisition of EMG data (measurement of the electrical activity of the jaw musculature 551) either at rest or in function.

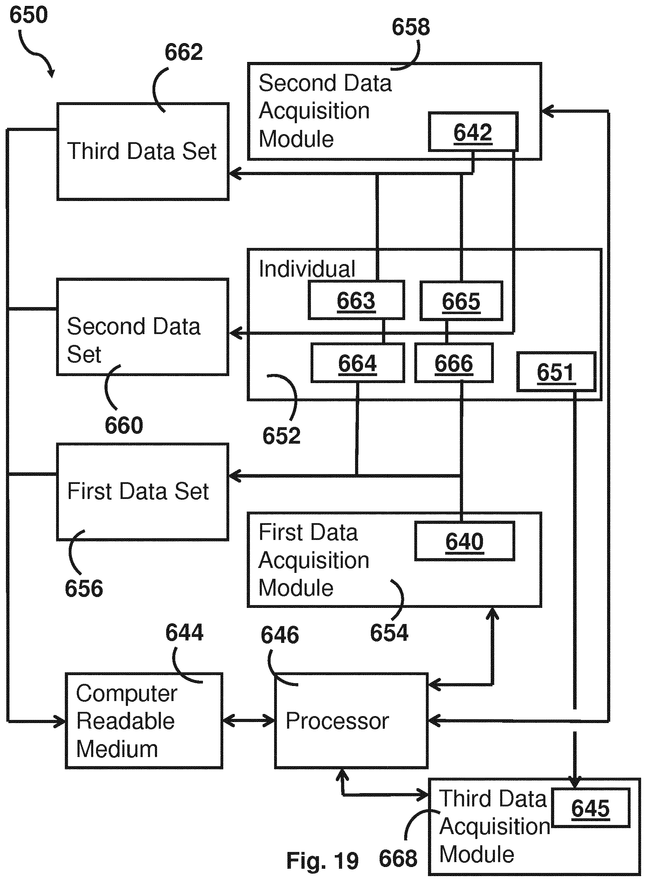

Fig. 19 is a schematic of a system 650 wherein the third data acquisition module 668 is in operative communication with the processor 646. The processor 646 may be configured to cause the second data acquisition module 658 to acquire the third data set 662 when one or more selected conditions are fulfilled. A selected condition may be that data acquired by the third data acquisition module 668 is indicative that the amount of energy used by the jaw musculature 651 to remain in position has reached a selected value, for example a minimum, which is indicative of the jaw musculature 651 being exhausted and the maxillo-mandibular relationship being at the rest position. Instructions for the processor 646 may be stored in the form of computer-readable code stored on the computer-readable medium 644. Coding of software including the instructions is within the scope of a person of ordinary skill in the art of computer programming given the present description. In some embodiments, where the third data acquisition module 668 is stabilized in the data acquisition position, the third data set 662 may be acquired without a clinician in the room during acquisition of the third data set 662. Where the method 110, or other methods wherein the third data set 662 includes data at maxillo-mandibular relationships other than the rest position, are practiced, a selected condition may be levels of energy usage by the jaw musculature 651 other than a level indicating that the maxillo-mandibular relationship is in the rest position.

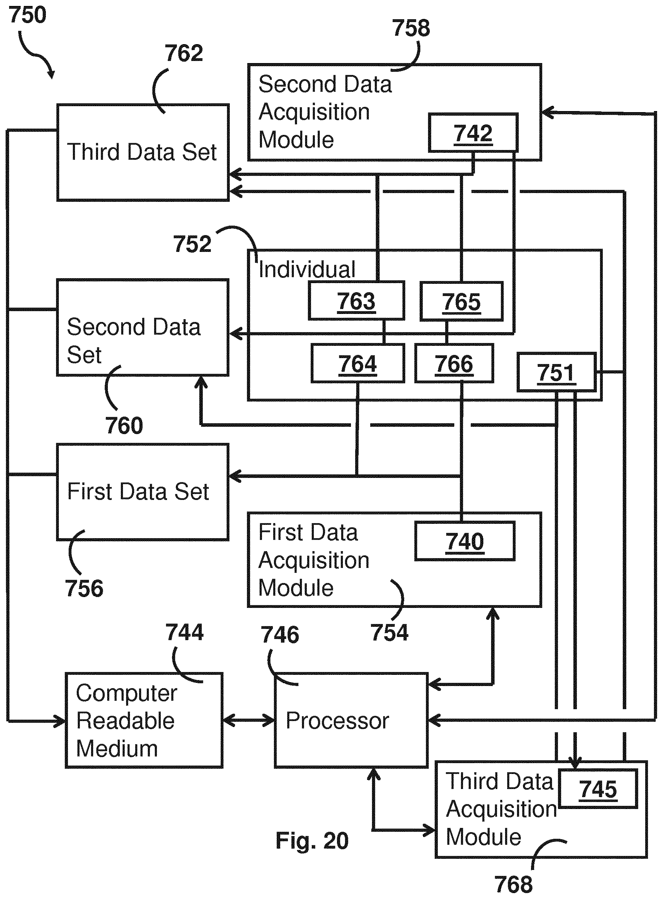

Fig. 20 is a schematic of a system 750 wherein the third data acquisition module 768 acquires data for the second data set 760, the third data set 762, or both. The third data set 762 may include data of both the position of the mandible 765 acquired by the second data acquisition module 758 and data of energy usage by the jaw musculature 751 acquired by the third data acquisition module 768. In an embodiment, the second data acquisition module 758 and the third data acquisition module 768 may acquire the third data set 762 simultaneously. In an embodiment, the second data set 760 may also include data of energy usage by the jaw musculature 751. In an embodiment, the data of energy usage by the jaw musculature 751 is acquired in real time, for example as a function of maxillo-mandibular relationship; this embodiment of the system 750 may have particular application to the method 110 to acquire the third data set 762 at maxillo-mandibular relationships other than the rest position.

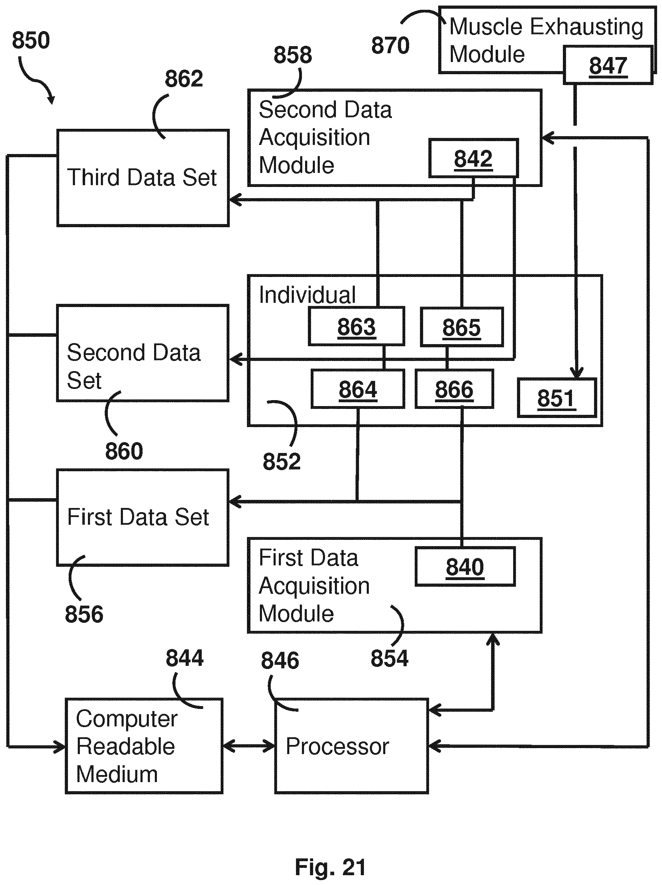

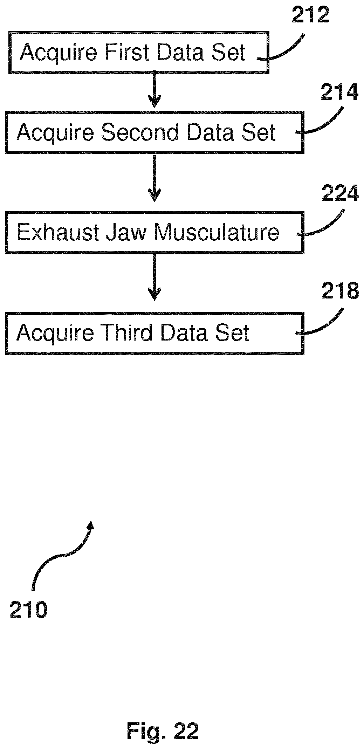

Fig. 21 is a schematic of an embodiment of a system 850 including a muscle exhausting module 870. Fig. 22 is a flow chart of a method 210 including exhausting the individual's jaw musculature 224. The muscle exhausting module 870 includes a muscle exhaustion apparatus 847, for example an electrode. When the jaw musculature 851 is exhausted, a minimal amount of energy is used to maintain the position of the mandible 865 and the maxillo-mandibular relationship is in the rest position.

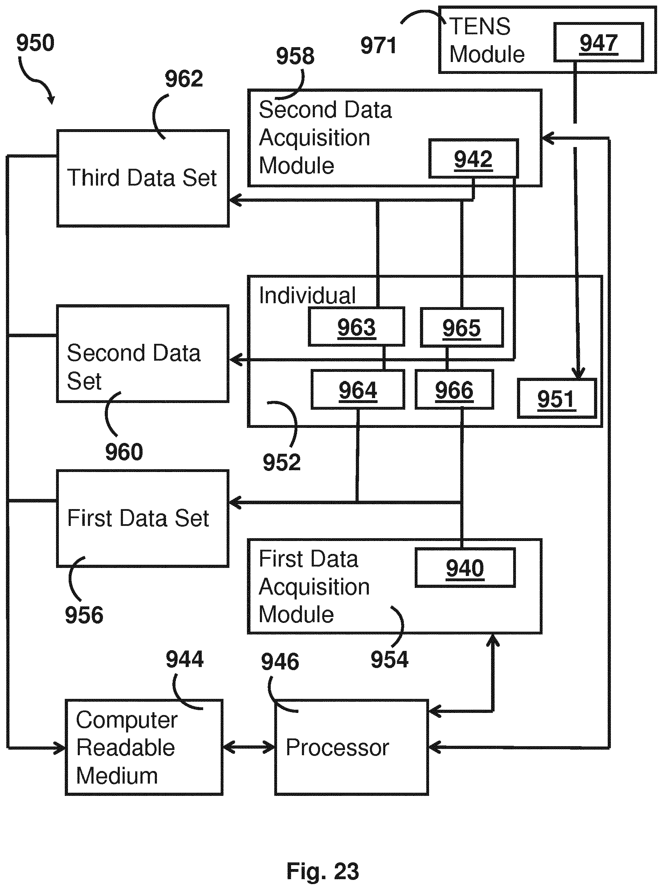

Fig. 23 is a schematic of a system 950 wherein the muscle exhausting module is a transcutaneous electrical nerve stimulation ("**TENS**") module 971. TENS is used to stimulate the jaw musculature 951 to exhaustion.

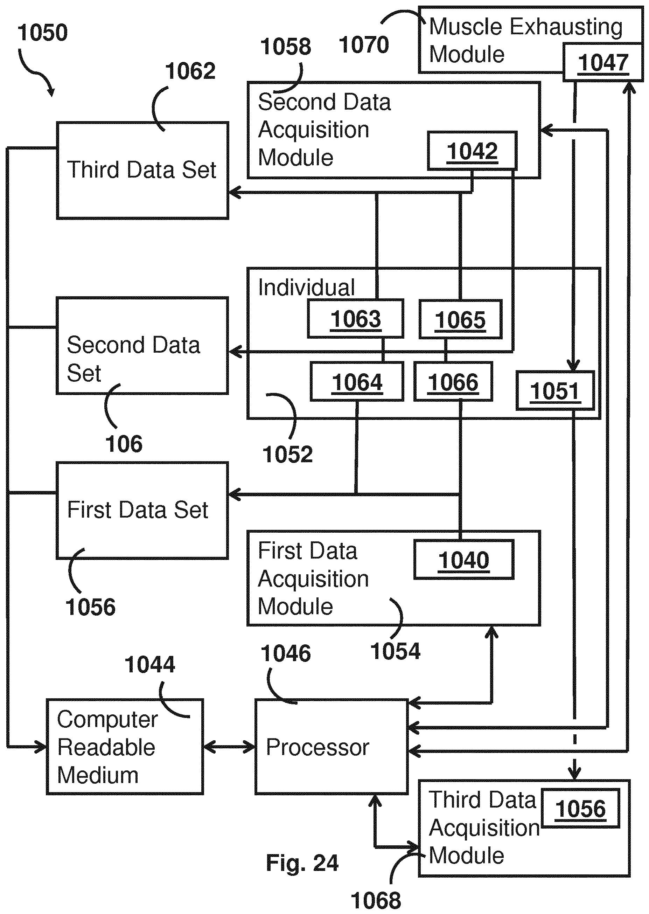

Fig. 24 is a schematic of a system 1050 further including the muscle exhausting module 1070 and the third data acquisition module 1068. The processor 1046 is in operative communication with the third data acquisition module 1068 and with the muscle exhausting module 1070. The muscle exhausting module 1070 exhausts the jaw musculature 1051 and the third data acquisition module 1068 acquires data indicative that the jaw musculature 1051 is exhausted. The processor 1046 may be configured to cause the second data acquisition module 1058 to acquire the third data set 1062 when the third data acquisition module 1068 acquires data indicating that the jaw musculature 1051 is exhausted by the muscle exhausting module 1070. The processor 1046 may be configured to cause the muscle exhausting module 1070 to exhaust the jaw musculature 1051. Instructions for the processor may be stored in the form of computer-readable code stored on the computer-readable medium 1044. Coding of software including the instructions is within the scope of a person of ordinary skill in the art of computer programming given the present description.

In the system 1050, it is unnecessary for the third data set 1062 to be interpreted in real time by a clinician, as the muscle exhausting module 1070 will exhaust the jaw musculature 1051 and the third data acquisition module 1068 will cause the second data acquisition module 1058 to acquire the third data set 1062. In some embodiments, where the third data acquisition module 1068 is stabilized in the data acquisition position, the third data set 1062 may be acquired without a clinician in the room during acquisition of the third data set 1062.

### Existing Dental Features

The methods and systems disclosed herein may be applied to an individual lacking any existing dental features (i.e. no teeth on either the maxillary arch or mandibular arch, where the maxillary arch is a residual ridge and palate only, and the mandibular arch is a residual ridge only). The methods and systems may also be applied to an individual may have existing dental features. The existing dental features are exemplified by prosthetics but may also include, for example, natural dentition. Where the dental features are removable prosthetics, they may remain in place during acquisition of the second data set and the third data set. The dental features may be accounted for when applying the second data set and the third data set to an application, for example preparing a 3D model to plan dental treatment.

### Preparing a 3D Model

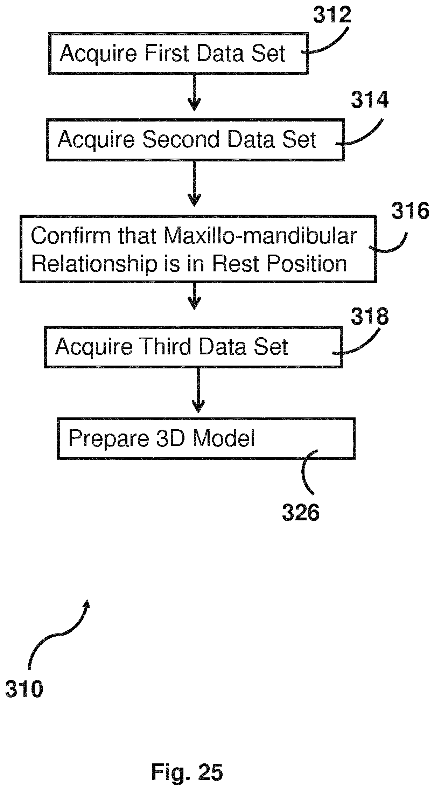

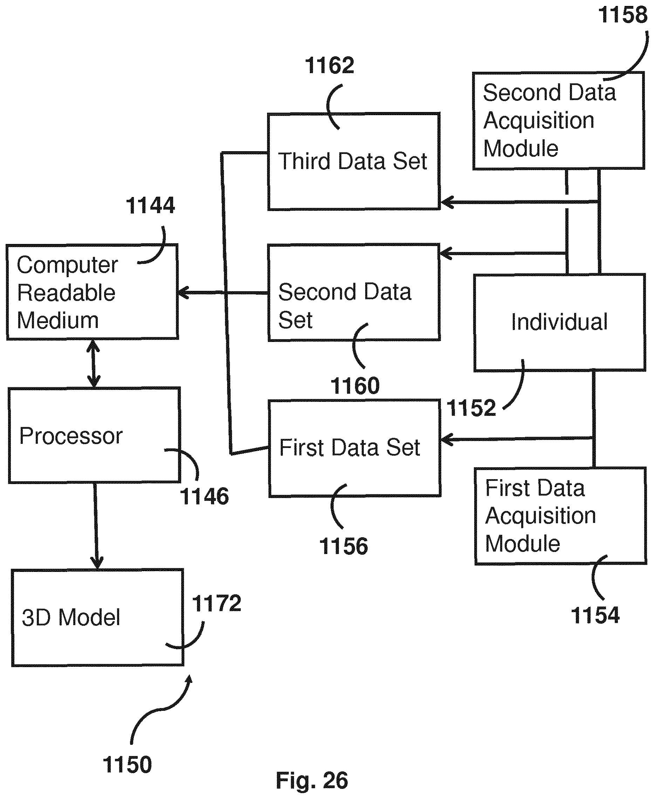

Fig. 25 is a flowchart of a method 310 including preparing a 3D model from the first data set, the second data set, and the third data set 326. Fig. 26 is a schematic of a system 1150 including a 3D model 1172 prepared from the first data set 1156, the second data set 1160, and the third data set 1162. The 3D model 1172 is a model of the head of the individual 1152, including the maxilla 1163, the maxillary arch 1164, the mandible 1165, and the mandibular arch 1166.

The first data set 1156 facilitates modeling of the maxillary arch 1164 and the mandibular arch 1166. The third data set 1162 facilitates modeling of the maxilla 1163 and the mandible 1165 at the maxillo-mandibular relationship of the rest position. The rest position of the 3D model 1172 is thus specific to the individual 1152 and accurately reflects the rest position of the individual 1152. The relative positions of the maxillary arch 1164 and the maxilla 1163 will remain constant at any maxillo-mandibular relationship, while the relative positions of the mandibular arch 1166 and mandible 1165 will similarly remain constant at any maxillo-mandibular relationship. Thus, the second data 1160 set provides a basis upon which to relate the first data set 1156 to the third data set 1162.

In the 3D model 1172, the model of the mandible 1165 may be hinged, translated, extended, and intended from the rest position. The rest position may thus be the reference position of the mandible 1165 from which diagnostic and therapeutic decisions are made.

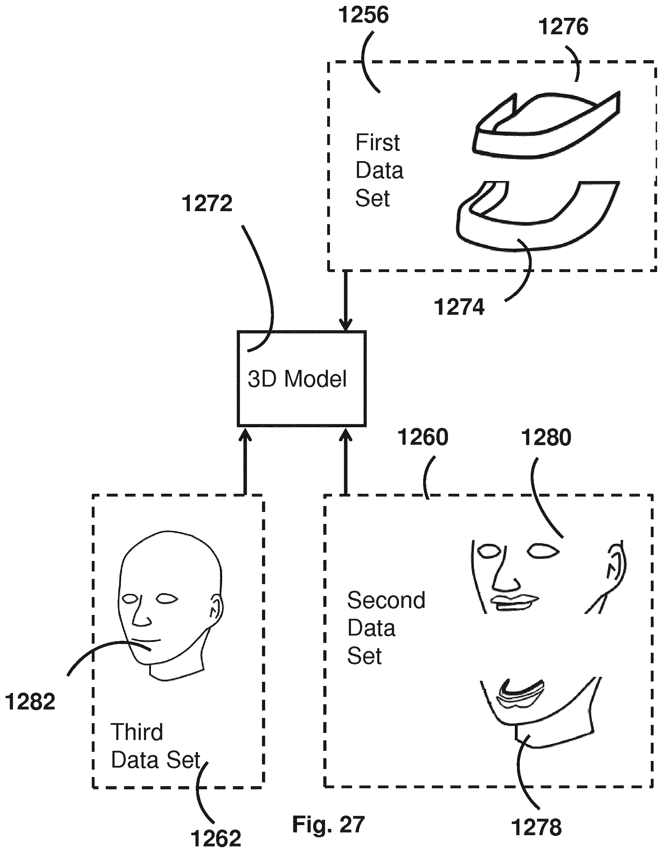

Fig. 27 is a schematic of a 3D model 1272 based on the data acquired by the method 10. The first data set 1256 allows preparation of a mandibular arch rendering 1274 and a maxillary arch rendering 1276. The second data set 1260 allows preparation of a mandibular rendering 1278 and a maxillary rendering 1280. The third data set 1262 allows preparation of a rest position rendering 1282. The model 1272 is prepared from the combined mandibular arch rendering 1274, maxillary arch rendering 1276, mandibular rendering 1278, maxillary rendering 1280, and rest position rendering 1282. The first data set 1256 is acquired by an optical technique, for example using the intra-oral scanner 155. The second data set 1260 and the third data set 1262 are each acquired by optical techniques, for example using the extra-oral scanner 259.

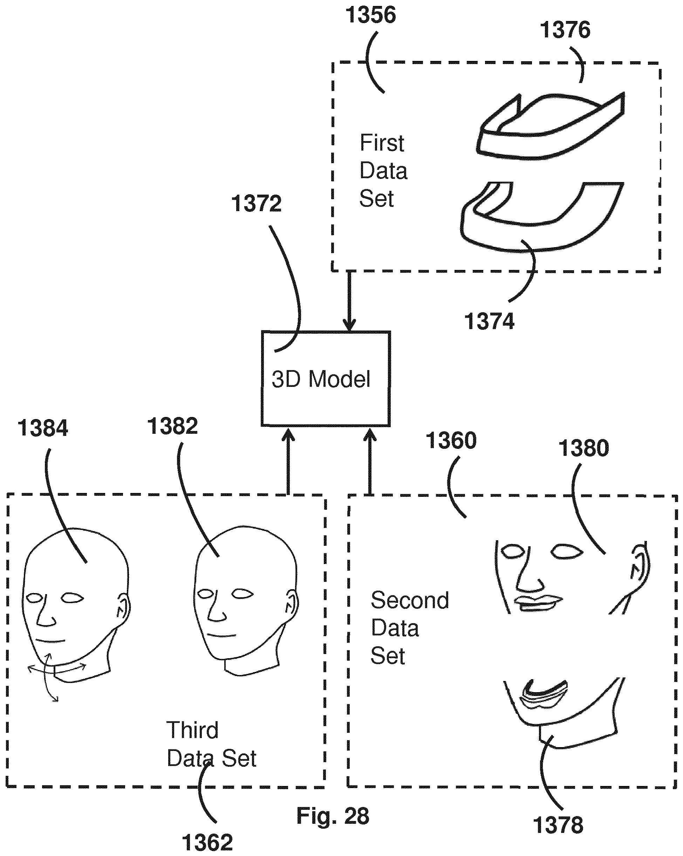

Fig. 28 is a schematic of a 3D model 1372 based on data acquired by the method 110. The third data set 1362 allows preparation of an other maxillo-mandibular relationship rendering 1384. The model 1372 is prepared from the combined mandibular arch rendering 1374, maxillary arch rendering 1376, mandibular rendering 1378, maxillary rendering 1380, rest position rendering 1382, and other maxillo-mandibular relationship rendering 1384. The first data set 1356 is acquired by an optical technique, for example using the intra-oral scanner 155. The second data set 1360 and the third data set 1362 are each acquired by optical techniques, for example using the extra-oral scanner 259.

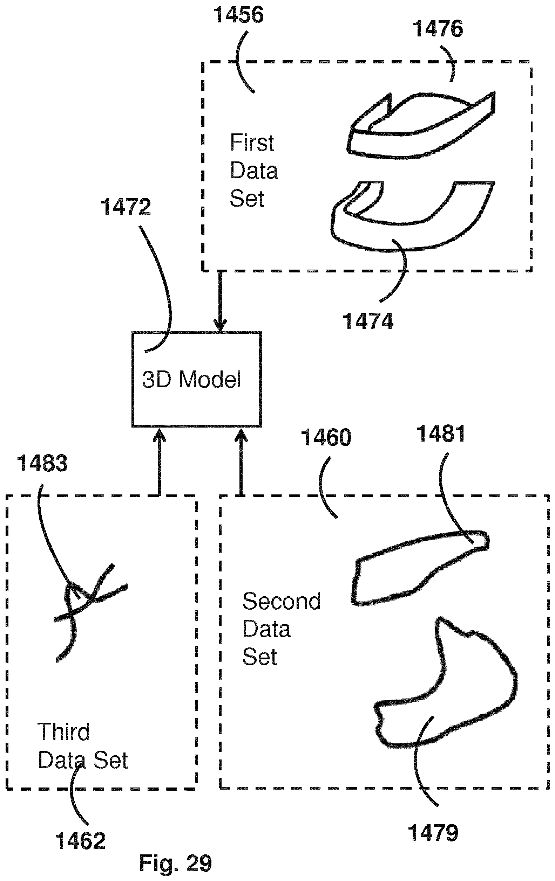

Fig. 29 is a schematic of a 3D model 1472 based on the data acquired by the method 10. The first data set 1456 allows preparation of a mandibular arch rendering 1474 and a maxillary arch rendering 1476. The second data set 1460 allows preparation of a mandibular rendering 1479 and a maxillary rendering 1481. The third data set 1462 allows preparation of a rest position rendering 1483. The model 1472 is prepared from the combined mandibular arch rendering 1474, maxillary arch rendering 1476, mandibular rendering 1479, maxillary rendering 1481, and rest position rendering 1483. The first data set 1456 is acquired by an optical technique, for example using the intra-oral scanner 155. The second data set 1460 and the third data set 1462 are each acquired by sonography techniques, for example using the 3D sonography module 361.

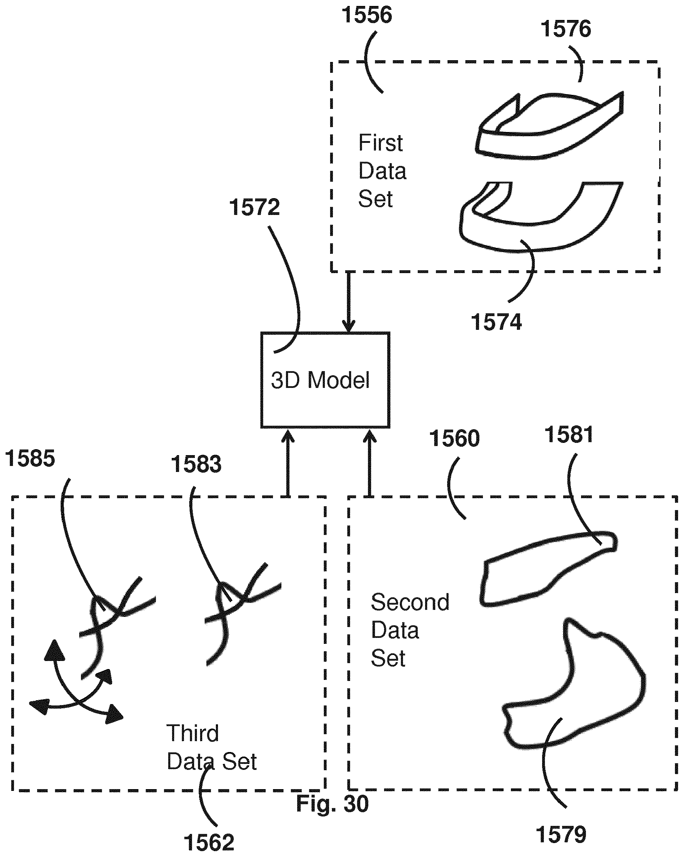

Fig. 30 is a schematic of a 3D model 1572 based on data acquired by the method 110. The third data set 1562 allows preparation of a other maxillo-mandibular relationship rendering 1584. The model 1572 is prepared from the combined mandibular arch rendering 1574, maxillary arch rendering 1576, mandibular rendering 1579, maxillary rendering 1581, rest position rendering 1583, and the other maxillo-mandibular relationship rendering 1585. The first data set 1556 is acquired by an optical technique, for example using the intra-oral scanner 155. The second data set 1560 and the third data set 1562 are each acquired by sonography techniques, for example using the 3D sonography module 361.

Figs. 27 to 30 show combinations of data acquired by the intra-oral scanner 155 used in combination with either the extra-oral 3D scanner 259 or the 3D sonography module 361. However, many other combinations are possible. For example, the intra-oral scanner 155 could be used in combination with the extra-oral 3D scanner 259 and the 3D sonography module 361. Alternatively, other examples of the first data acquisition module 54 and the second data acquisition module 58 may be used in combination with the above examples, in combination with each other, or both.

### Using the 3D Model

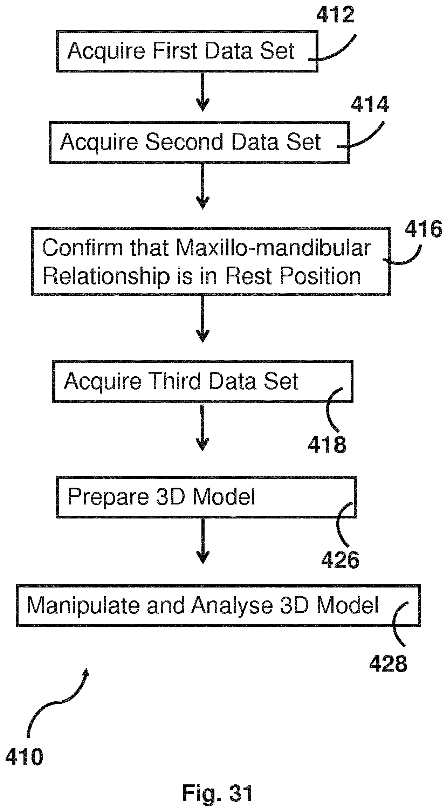

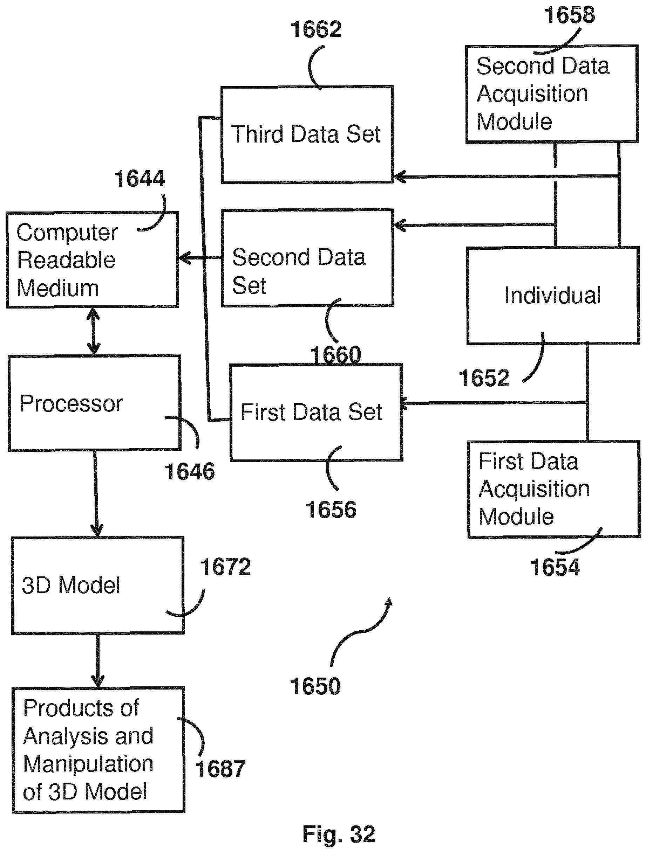

Fig. 31 is a flowchart of a method 410 including manipulations and analysis of the 3D model 428 resulting from preparing the 3D model 426. Fig. 32 is a schematic of a system 1650 including products of manipulation and analysis of the 3D model 1687. Manipulation and analysis of the 3D model 1672 may include extrapolating to maxillo-mandibular relationships other than the rest position and measuring relationships between features of the 3D model 1672.

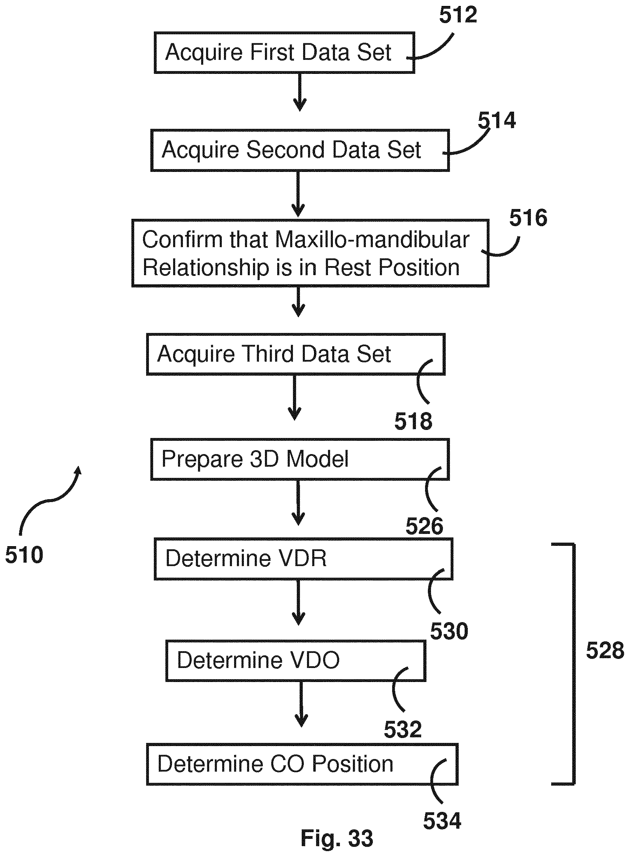

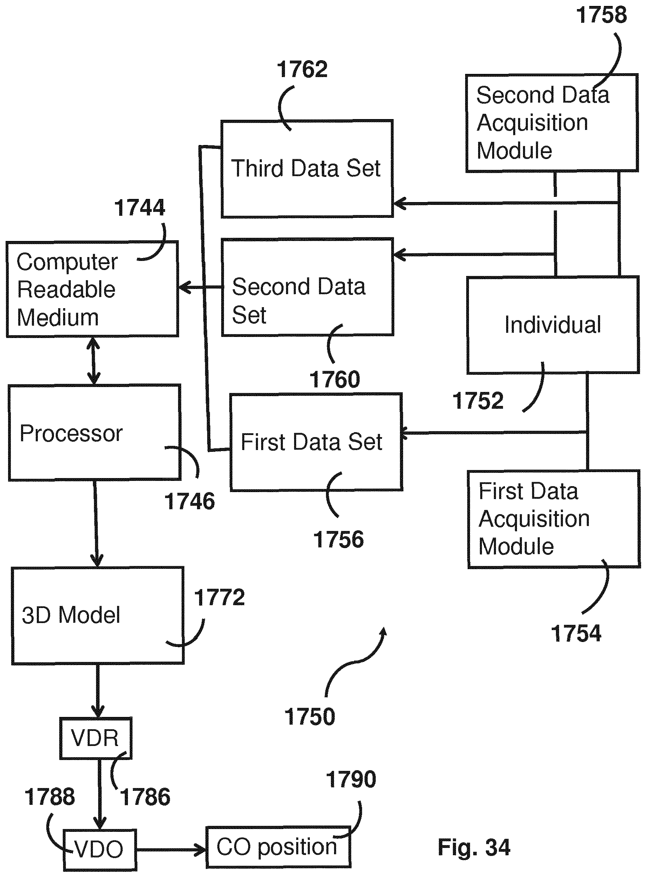

Fig. 33 is a flowchart of a method 510 wherein manipulating and analyzing the 3D model 528 resulting from preparing the 3D model 526 includes determining a vertical dimension of rest ("**VDR**") 530, determining a vertical dimension of occlusion ("**VDO**") 532, and determining a centric occlusion ("**CO**") position 534. Fig. 34 is a schematic of a system 1750 wherein the products of manipulation and analysis of the 3D model 1787 include a VDR 1786, a VDO 1788, and a CO position 1790.

In the system 1750 and method 510, the 3D model 1772 is used to extrapolate the CO position 1790. At the rest position, the VDR 1786 may be measured between a first arbitrary point on the maxilla 1763 and second arbitrary point on the mandible 1765. The model 1772 has a VDO 1788 when the maxillo-mandibular relationship is in the CO position 1790. The rest position is typically down and forward of the CO position 1790 and the VDO 1788 is typically between about 1 and about 4 mm less than the VDR 1786 (measuring from the same arbitrary points).

In the 3D model 1772, the maxillo-mandibular relationship is in the rest position, which serves as a reference point that may be used to extrapolate the CO position 1790. The VDR 1786 is determined. From the VDR 1786, the VDO 1788 is estimated. The VDO 1788 provides an estimated CO position 1790 for the individual. The maxillo-mandibular relationship for the individual 1752 at the CO position 1790 is a reference point from which the particular features of the individual 1752 may be considered to when preparing dental appliances, for example dentures, for the individual 1752.

In an embodiment, the vertical dimension in the 3D model 1772 is closed by between about 1 and about 4 mm from the VDR 1786 to place the model of the mandible 1765 at an estimated VDO from the model of the maxilla 1763.

In an embodiment, the vertical dimension in the 3D model is closed by between about 1 and about 2 mm from the VDR 1786 to place the model of the mandible 1765 at an estimated VDO from the model of the maxilla 1763.

### Potential Advantages

Through use of a 3D model of the individual's head based on empirical data of the rest position, some potential sources of error that may be present when designing dentures are avoided:
- manipulation of the individual's lips to expose an inter-arch space;
- maintenance by the individual of a consistent maxillo-mandibular relationship while bite registration material is injected, cured, and hardened;
- use of impression material (for example gypsum, alginates, polyvinylsiloxanes, or polyethers), which may have a degree of dimensional instability, to form an impression;
- suspension of dental models in place with gypsum, which shrinks, incurring a degree of dimensional instability, as it cures and hardens; and
- use of occlusal rims or bite blocks resting on the individual's arches, which may be compressed during loading, for example, during a bite registration.

An additional source of error may be mitigated by remote acquisition of the second and third data sets. Some individuals experience a degree of dental fear, which may range from mild to severe. Some individuals experience odontophobia, which may make them fearful of receiving dental treatment to the point that they avoid dental care. The resulting stress and anxiety may affect the individual's ability to maintain a jaw position, for example a rest position. This may complicate efforts to register a bite by injecting bite registration material and allowing it to cure and harden. This stress and anxiety experienced by an individual may be exacerbated by close proximity to a dental clinical. While registering a bite, a clinician may be in contact with the individual for prolonged periods of time, sometimes including during curing and hardening of bit registration material. By removing all dental clinicians and observers from the environment, these effects can be mitigated and a more accurate bite may be registered. Accordingly, in some embodiments disclosed herein, the individual is left alone in a room during at least part of the time when the third data sets is acquired.

### Examples Only

In the preceding description, for purposes of explanation, numerous details are set forth in order to provide a thorough understanding of the embodiments. However, it will be apparent to one skilled in the art that these specific details are not required. In other instances, well-known electrical structures and circuits are shown in block diagram form in order not to obscure the understanding. For example, specific details are not provided as to whether the embodiments described herein are implemented as a software routine, hardware circuit, firmware, or a combination thereof.

Embodiments of the disclosure can be represented as a computer program product stored in a machine-readable medium (also referred to as a computer-readable medium, a processor-readable medium, or a computer usable medium having a computer-readable program code embodied therein). The machine-readable medium can be any suitable tangible, non-transitory medium, including magnetic, optical, or electrical storage medium including a diskette, compact disk read only memory (CD-ROM), memory device (volatile or non-volatile), including a solid state storage device, removable USB solid state storage (e.g. USB flash drive), solid state drive, secure digital (SD) memory device, mini SD memory card, micro SD memory card, hard disk drive, hybrid drive, or similar storage mechanism. The machine-readable medium can contain various sets of instructions, code sequences, configuration information, or other data, which, when executed, cause a processor to perform a method according to an embodiment of the disclosure. Those of ordinary skill in the art will appreciate that other instructions and operations necessary to implement the described implementations can also be stored on the machine-readable medium. The instructions stored on the machine-readable medium can be executed by a processor or other suitable processing device, and can interface with circuitry to perform the described tasks.

The above-described embodiments are intended to be examples only. Alterations, modifications and variations can be effected to the particular embodiments by those of skill in the art without departing from the scope, which is defined solely by the claims appended hereto.

## Claims

1. A method (10) of acquiring data from an individual (52) for preparing a 3D model (1172) of the individual (52), the method comprising:
acquiring (12) a first data set (56) to facilitate structural modeling of at least a portion of a maxillary arch (64) of the individual (52) and at least a portion of a mandibular arch (66) of the individual (52);
acquiring (14) a second data set (60) to facilitate structural modeling of at least a portion of a maxilla (63) of the individual (52) and at least a portion of the maxillary arch (64) for relating the maxilla (63) to the maxillary arch (64), and of at least a portion of a mandible (65) of the individual (52) and at least a portion of the mandibular arch (66) for relating the mandible (65) to the mandibular arch (66);
exhausting the jaw musculature (451) of the individual (52) for confirming (16) that the maxillo-mandibular relationship is at the physiological rest position; and
acquiring (18) a third data set (62) to facilitate structural modeling of at least a portion of the maxilla (63) and at least a portion of the mandible (65) when the maxillo-mandibular relationship is at the physiological rest position.

2. The method of claim 1 wherein exhausting the jaw musculature (451) comprises stimulating the jaw musculature (451) to exhaustion.

3. The method of claim 1 further comprising monitoring energy usage by jaw musculature (451) for confirming (16) that a maxillo-mandibular relationship of the individual (52) is at a physiological rest position, and wherein the third data set (62) is acquired when a selected energy usage value is monitored.

4. The method of claim 1 further comprising monitoring energy usage by jaw musculature (451) for confirming (16) that a maxillo-mandibular relationship of the individual (52) is at a physiological rest position, and wherein energy usage is included in the third data set (62).

5. The method of claim 1 further comprising combining the first data set (56), the second data set (60), and the third data set (62) to render an articulatable 3D model (1172) of the individual (52) in the physiological rest position.

6. The method of claim 5 further comprising determining a vertical dimension of rest for a maxillo-mandibular relationship of the articulatable 3D model (1172) and positioning a mandible of the articulatable 3D model (1172) at a vertical dimension of between 1 and 4 mm vertically closed from the vertical dimension of rest to provide an estimated centric occlusion position.

7. The method of claim 6 further comprising preparing a dental appliance based on the estimated centric occlusion position.

8. A system (50) for acquiring data for preparing a 3D model (1172) from an individual (52) comprising:
a first data acquisition module (54) comprising a first sensor (40) for acquiring a first data set (56) of a maxillary arch (64) of the individual (52) and of a mandibular arch (66) of the individual (52);
a second data acquisition module (58) comprising a second sensor (42) for acquiring a second data set (60) of at least a portion of a maxilla (63) of the individual (52) and at least a portion of the maxillary arch (64) for relating the maxilla (63) to the maxillary arch (64), and of at least a portion of a mandible (65) of the individual (52) and at least a portion of the mandibular arch (66) for relating the mandible (65) to the mandibular arch (66), and for acquiring a third data set (62) of at least a portion of the maxilla (63) and at least a portion of the mandible (65) when a maxillo-mandibular relationship of the individual (52) is at a physiological rest position;
a processor (46) in operative communication with the first data acquisition module (54) and the second data acquisition module (58) for controlling the first data acquisition module (54) and the second data acquisition module (58); and
a muscle exhaustion module (870) for exhausting the jaw musculature (451).

9. The system of claim 8 wherein the processor (46) is in operative communication with the muscle exhaustion module (870) for controlling and receiving feedback from the muscle exhaustion module (870).

10. The system of either of claims 8 or 9 wherein the second data acquisition module (58) is stabilized in a data acquisition position where the third data set (62) may be acquired.

11. The system of claim 10 wherein the processor (46) is further configured to cause the second data acquisition module (58) to acquire the third data set (60).

12. The system of claim 10 wherein the second data set (60) may be acquired from the data acquisition position, and wherein the processor (46) is further configured to cause the second data acquisition module (58) to acquire the second data set (60).

13. The system of claim 9 further comprising a third data acquisition module (468) comprising a third sensor (345) for monitoring energy usage of jaw musculature (451) of the individual (52).

14. The system of claim 13 wherein the third data acquisition module (468) is in operative communication with the processor (46) and the processor (46) is configured to cause the second data acquisition module (58) to acquire the third data set (62) when energy usage of the jaw musculature (451) is at a minimum energy usage indicative of the jaw musculature (451) being exhausted and the maxillo-mandibular relationship being at the rest position.

15. The system of claim 13 wherein the third data set (62) is further acquired by the third data acquisition module (468).

16. A computer readable medium (44) comprising instructions for causing a processor (46) to perform the method of any of claims 1 to 7.

## Patentansprüche

1. Verfahren (10) zum Erfassen von Daten von einer Person (52) zur Herstellung eines 3D-Modells (1172) der Person (52), wobei das Verfahren umfasst:
Erfassen (12) eines ersten Datensatzes (56) zum Erleichtern einer strukturellen Modellierung mindestens eines Teil eines Oberkieferbogens (64) der Person (52) und mindestens eines Teils eines Unterkieferbogens (66) der Person (52);
Erfassen (14) eines zweiten Datensatzes (60) zum Erleichtern einer strukturellen Modellierung mindestens eines Teils eines Oberkiefers (63) der Person (52) und mindestens eines Teils des Oberkieferbogens (64) zur Zuordnung des Oberkiefers (63) zu dem Oberkieferbogen (64), und mindestens eines Teils eines Unterkiefers (65) der Person (52) und mindestens eines Teils des Unterkieferbogens (66) zur Zuordnung des Unterkiefers (65) zu dem Unterkieferbogen (66);
Erschöpfen der Kiefermuskulatur (451) der Person (52), um zu bestätigen (16), dass sich die Oberkiefer-Unterkiefer-Beziehung in der physiologischen Ruheposition befindet; und
Erfassen (18) eines dritten Datensatzes (62) zum Erleichtern einer strukturellen Modellierung mindestens eines Teils des Oberkiefers (63) und mindestens eines Teils des Unterkiefers (65), wenn sich die Oberkiefer-Unterkiefer-Beziehung in der physiologischen Ruheposition befindet.

2. Verfahren nach Anspruch 1, wobei das Erschöpfen der Kiefermuskulatur (451) ein Stimulieren der Kiefermuskulatur (451) zur Erschöpfung umfasst.

3. Verfahren nach Anspruch 1, ferner umfassend ein Überwachen eines Energieverbrauchs durch die Kiefermuskulatur (451), um zu bestätigen (16), dass sich eine Oberkiefer-Unterkiefer-Beziehung der Person (52) in einer physiologischen Ruheposition befindet, und wobei der dritte Datensatz (62) erfasst wird, wenn ein ausgewählter Energieverbrauchswert überwacht wird.

4. Verfahren nach Anspruch 1, ferner umfassend ein Überwachen eines Energieverbrauchs durch die Kiefermuskulatur (451), um zu bestätigen (16), dass sich eine Oberkiefer-Unterkiefer-Beziehung der Person (52) in einer physiologischen Ruheposition befindet, und wobei ein Energieverbrauch in dem dritten Datensatz (62) enthalten ist.

5. Verfahren nach Anspruch 1, ferner umfassend ein Kombinieren des ersten Datensatzes (56), des zweiten Datensatzes (60) und des dritten Datensatzes (62), um ein artikulierbares 3D-Modell (1172) der Person (52) in der physiologischen Ruheposition zu erstellen.

6. Verfahren nach Anspruch 5, ferner umfassend ein Bestimmen einer vertikalen Ruhe-Abmessung für eine Oberkiefer-Unterkiefer-Beziehung des artikulierbaren 3D-Modells (1172) und Positionieren eines Unterkiefers des artikulierbaren 3D-Modells (1172) an einer vertikalen Abmessung, die zwischen 1 und 4 mm vertikal verkürzt von der vertikalen Ruhe-Abmessung ist, um eine geschätzte zentrische Okklusionsposition bereitzustellen.

7. Verfahren nach Anspruch 6, ferner umfassend ein Herstellen einer Dentalvorrichtung basierend auf der geschätzten zentrischen Okklusionsposition.

8. System (50) zum Erfassen von Daten zur Herstellung eines 3D-Modells (1172) von einer Person (52), umfassend:
ein erstes Datenerfassungsmodul (54), umfassend einen ersten Sensor (40) zum Erfassen eines ersten Datensatzes (56) eines Oberkieferbogens (64) der Person (52) und eines Unterkieferbogens (66) der Person (52);
ein zweites Datenerfassungsmodul (58), umfassend einen zweiten Sensor (42) zum Erfassen eines zweiten Datensatzes (60) mindestens eines Teil eines Oberkiefers (63) der Person (52) und mindestens eines Teils des Oberkieferbogens (64) zur Zuordnung des Oberkiefers (63) zu dem Oberkieferbogen (64), und mindestens eines Teils eines Unterkiefers (65) der Person (52) und mindestens eines Teils des Unterkieferbogens (66) zur Zuordnung des Unterkiefers (65) zu dem Unterkieferbogen (66), und zum Erfassen eines dritten Datensatzes (62) mindestens eines Teils des Oberkiefers (63) und mindestens eines Teils des Unterkiefers (65), wenn sich eine Oberkiefer-Unterkiefer-Beziehung der Person (52) in einer physiologischen Ruheposition befindet;
einen Prozessor (46) in funktioneller Verbindung mit dem ersten Datenerfassungsmodul (54) und dem zweiten Datenerfassungsmodul (58) zur Steuerung des ersten Datenerfassungsmoduls (54) und des zweiten Datenerfassungsmoduls (58); und
ein Muskelerschöpfungsmodul (870) zum Erschöpfen der Kiefermuskulatur (451).

9. System nach Anspruch 8, wobei der Prozessor (46) in funktioneller Verbindung mit dem Muskelerschöpfungsmodul (870) steht, um eine Rückmeldung von dem Muskelerschöpfungsmodul (870) zu steuern und zu empfangen.

10. System nach Anspruch 8 oder 9, wobei das zweite Datenerfassungsmodul (58) in einer Datenerfassungsposition stabilisiert ist, in der der dritte Datensatz (62) erfasst werden kann.

11. System nach Anspruch 10, wobei der Prozessor (46) ferner konfiguriert ist, zu bewirken, dass das zweite Datenerfassungsmodul (58) den dritten Datensatz (60) erfasst.

12. System nach Anspruch 10, wobei der zweite Datensatz (60) von der Datenerfassungsposition erfasst werden kann, und wobei der Prozessor (46) ferner konfiguriert ist, zu bewirken, dass das zweite Datenerfassungsmodul (58) den zweiten Datensatz (60) erfasst.

13. System nach Anspruch 9, ferner umfassend ein drittes Datenerfassungsmodul (468), das einen dritten Sensor (345) zum Überwachen des Energieverbrauchs der Kiefermuskulatur (451) der Person (52) umfasst.

14. System nach Anspruch 13, wobei das dritte Datenerfassungsmodul (468) in funktioneller Verbindung mit dem Prozessor (46) steht und der Prozessor (46) konfiguriert ist, zu bewirken, dass das zweite Datenerfassungsmodul (58) den dritten Datensatz (62) erfasst, wenn ein Energieverbrauch der Kiefermuskulatur (451) bei einem minimalen Energieverbrauch ist, der angibt, dass die Kiefermuskulatur (451) erschöpft ist, und sich die Oberkiefer-Unterkiefer-Beziehung in der Ruheposition befindet.

15. System nach Anspruch 13, wobei der dritte Datensatz (62) ferner durch das dritte Datenerfassungsmodul (468) erfasst wird.

16. Computerlesbares Medium (44), das Anweisungen umfasst zum Bewirken, dass ein Prozessor (46) das Verfahren nach einem der Ansprüche 1 bis 7 durchführt.

## Revendications

1. Procédé (10) d'acquisition de données auprès d'un individu (52) pour préparer un modèle 3D (1172) de l'individu (52), le procédé comprenant :
l'acquisition (12) d'un premier jeu de données (56) pour faciliter une modélisation structurelle d'au moins une portion d'un arc maxillaire (64) de l'individu (52) et d'au moins une portion d'un arc mandibulaire (66) de l'individu (52) ;
l'acquisition (14) d'un deuxième jeu de données (60) pour faciliter une modélisation structurelle d'au moins une portion d'un maxillaire (63) de l'individu (52) et d'au moins une portion de l'arc maxillaire (64) pour mettre le maxillaire (63) en relation avec l'arc maxillaire (64), et d'au moins une portion d'une mandibule (65) de l'individu (52) et d'au moins une portion de l'arc mandibulaire (66) pour mettre la mandibule (65) en relation avec l'arc mandibulaire (66) ;
l'épuisement de la musculature de mâchoire (451) de l'individu (52) pour confirmer (16) que la relation maxillo-mandibulaire est à la position de repos physiologique ; et
l'acquisition (18) d'un troisième jeu de données (62) pour faciliter une modélisation structurelle d'au moins une portion du maxillaire (63) et d'au moins une portion de la mandibule (65) lorsque la relation maxillo-mandibulaire est à la position de repos physiologique.

2. Procédé selon la revendication 1, dans lequel l'épuisement de la musculature de mâchoire (451) comprend la stimulation de la musculature de mâchoire (451) jusqu'à épuisement.

3. Procédé selon la revendication 1, comprenant en outre la surveillance d'une utilisation d'énergie par la musculature de mâchoire (451) pour confirmer (16) qu'une relation maxillo-mandibulaire de l'individu (52) est à une position de repos physiologique, et dans lequel le troisième jeu de données (62) est acquis lorsqu'une valeur d'utilisation d'énergie sélectionnée est surveillée.

4. Procédé selon la revendication 1, comprenant en outre la surveillance d'une utilisation d'énergie par la musculature de mâchoire (451) pour confirmer (16) qu'une relation maxillo-mandibulaire de l'individu (52) est à une position de repos physiologique, et dans lequel l'utilisation d'énergie est incluse dans le troisième jeu de données (62).

5. Procédé selon la revendication 1, comprenant en outre la combinaison du premier jeu de données (56), du deuxième jeu de données (60), et du troisième jeu de données (62) pour rendre un modèle 3D articulable (1172) de l'individu (52) à la position de repos physiologique.

6. Procédé selon la revendication 5, comprenant en outre la détermination d'une dimension verticale de repos pour une relation maxillo-mandibulaire du modèle 3D articulable (1172) et le positionnement d'une mandibule du modèle 3D articulable (1172) à une dimension verticale entre 1 et 4 mm verticalement fermée depuis la dimension verticale de repos pour fournir une position d'occlusion centrique estimée.

7. Procédé selon la revendication 6, comprenant en outre la préparation d'un appareil dentaire sur la base de la position d'occlusion centrique estimée.

8. Système (50) d'acquisition de données pour préparer un modèle 3D (1172) d'un individu (52) comprenant :
un premier module d'acquisition de données (54) comprenant un premier capteur (40) pour acquérir un premier jeu de données (56) d'un arc maxillaire (64) de l'individu (52) et d'un arc mandibulaire (66) de l'individu (52) ;
un deuxième module d'acquisition de données (58) comprenant un deuxième capteur (42) pour acquérir un deuxième jeu de données (60) d'au moins une portion d'un maxillaire (63) de l'individu (52) et d'au moins une portion de l'arc maxillaire (64) pour mettre le maxillaire (63) en relation avec l'arc maxillaire (64), et d'au moins une portion d'une mandibule (65) de l'individu (52) et d'au moins une portion de l'arc mandibulaire (66) pour mettre la mandibule (65) en relation avec l'arc mandibulaire (66), et pour acquérir un troisième jeu de données (62) d'au moins une portion du maxillaire (63) et d'au moins une portion de la mandibule (65) lorsqu'une relation maxillo-mandibulaire de l'individu (52) est à une position de repos physiologique ;
un processeur (46) en communication opérationnelle avec le premier module d'acquisition de données (54) et le deuxième module d'acquisition de données (58) pour commander le premier module d'acquisition de données (54) et le deuxième module d'acquisition de données (58) ; et
un module d'épuisement musculaire (870) pour épuiser la musculature de mâchoire (451).

9. Système selon la revendication 8, dans lequel le processeur (46) est en communication opérationnelle avec le module d'épuisement musculaire (870) pour commander et recevoir une rétroaction du module d'épuisement musculaire (870).

10. Système selon la revendication 8 ou 9, dans lequel le deuxième module d'acquisition de données (58) est stabilisé à une position d'acquisition de données à laquelle le troisième jeu de données (62) peut être acquis.

11. Système selon la revendication 10, dans lequel le processeur (46) est en outre configuré pour amener le deuxième module d'acquisition de données (58) à acquérir le troisième jeu de données (60).

12. Système selon la revendication 10, dans lequel le deuxième jeu de données (60) peut être acquis à partir de la position d'acquisition de données, et dans lequel le processeur (46) est en outre configuré pour amener le deuxième module d'acquisition de données (58) à acquérir le deuxième jeu de données (60).

13. Système selon la revendication 9, comprenant en outre un troisième module d'acquisition de données (468) comprenant un troisième capteur (345) pour surveiller une utilisation d'énergie d'une musculature de mâchoire (451) de l'individu (52).

14. Système selon la revendication 13, dans lequel le troisième module d'acquisition de données (468) est en communication opérationnelle avec le processeur (46) et le processeur (46) est configuré pour amener le deuxième module d'acquisition de données (58) à acquérir le troisième jeu de données (62) lorsqu'une utilisation d'énergie de la musculature de mâchoire (451) est à une utilisation d'énergie minimale indiquant que la musculature de mâchoire (451) est épuisée et que la relation maxillo-mandibulaire est à la position de repos.

15. Système selon la revendication 13, dans lequel le troisième jeu de données (62) est en outre acquis par le troisième module d'acquisition de données (468).

16. Support lisible par ordinateur (44) comprenant des instructions pour amener un processeur (46) à effectuer le procédé selon l'une quelconque des revendications 1 à 7.
